# EUROPEAN PATENT SPECIFICATION

(11) **EP 1 095 053 B1**
(45) Date of publication and mention of the grant of the patent: **11.12.2002**
(21) Application number: 99933033.5
(22) Date of filing: 13.07.1999
(51) Int. Cl.: C07H 21/00, C12Q 1/68, G01N 33/58, G01N 33/68

(54) **ARYLSULFONE LINKERS FOR MASS SPECTROMETRIC ANALYSIS**
ARYLSULFON-LINKER ZUR MASSENSPEKTROMETRISCHEN ANALYSE
LIANTS A BASE D'ARYLSULFONE POUR ANALYSE PAR SPECTROMETRIE DE MASSE

(30) Priority: 13.07.1998 GB 9815163
(43) Date of publication of application: 02.05.2001
(73) Proprietor: Xzillion GmbH & CO.KG, 65926 Frankfurt am Main (DE)
(72) Inventor: SCHMIDT, Günter, Cambs PE17 2BQ (GB); THOMPSON, Andrew Hugin, Alloway, Ayr KA7 4RH (GB); JOHNSTONE, Robert, Alexander, Walker, Bebington L63 9LD (GB)
(74) Representative: Daniels, Jeffrey Nicholas
(86) International application number: GB9902257
(87) International publication number: WO00002895

(56) References cited:
- EP-A- 0 596 355
- WO-A-94/13319
- WO-A-97/27327
- US-A- 5 118 605
- US-A- 5 216 141
- F BERGMANN, W PFEIDERER: "21. Nucleotides. Part XLI. The 2-Dansylethoxycarbonyl (=2-{[5-(Dimethylamino)naphthalen-1-yl]sul fonyl}ethoxycarbonyl; Dnseoc) Group for Protection of the 5'-Hydroxy Function in Oligodeoxribonucleotide Synthesis" HELVETICA CHIMICA ACTA, vol. 77, 1994, pages 203-215, XP002122219
- F BERGMANN, W PFEIDERER: "92. Nucleotides. Part XLIII. Solid-Phase Synthesis of Oligonucleotides Using the 2-Dansylethoxycarbonyl (=2-{[5-(Dimethylamino)naphthalen-1-yl]sul fonyl}ethoxycarbonyl; Dnseoc) Group for 5'-Hydroxy Protection" HELVETICA CHIMICA ACTA, vol. 77, 1994, pages 988-998, XP002122220
- M R LEWIS, J E SHIVELY: "Maleimidocysteineamido-DOTA Derivatives: New Reagents for Radiometal Chelate Conjugation to Antibody Sulfhydryl Groups Undergo pH-Dependent Cleavage Reactions" BIOCONJUGATE CHEMISTRY, vol. 9, no. 1, January 1998 (1998-01), pages 72-86, XP002122221

## Description

This invention relates to methods of labelling analytes with markers that are cleavably detachable from their associated analytes and that are detectable by mass spectrometry. Specifically this invention refers to improved methods of detaching mass labels from their associated proteins, nucleic acids or other molecules of interest.

Cleavable mass labels have a number of advantages over other methods of detecting analytes with labels. In the analysis of DNA, commercially favoured systems are based on fluorescent labelling of DNA. Fluorescent labelling schemes permit the labelling of a relatively small number of molecules simultaneously, in which typically four labels are used simultaneously and possibly up to eight. However, the costs of the detection apparatus and the difficulties in analysing the resultant signals limit the number of labels that can be used simultaneously in a fluorescence detection scheme. An advantage of using mass labels is the possibility of making large numbers of labels, which can be discretely resolved in a mass spectrometer thereby allowing similar numbers of distinct molecular species to be labelled simultaneously. A feature of the use of cleavable mass labels disclosed is the need for linker groups that covalently link a mass marker to its corresponding nucleic acid whilst, at the same time being easy to detach from the analyte so as to permit detection of the mass marker.

PCT/GB98/00127 describes arrays of nucleic acid probes covalently attached to cleavable labels that are detectable by mass spectrometry which identify the sequence of a covalently linked nucleic acid probe. These mass labels have a number of advantages over other methods of analysing nucleic acids. The labelled probes of this application have the structure Nu-L-M where Nu is a nucleic acid covalently linked to L, a cleavable linker, covalently linked to M, a mass label. Preferred cleavable linkers in this application cleave within the ion source of the mass spectrometer, specifically within electrospray ion sources. Preferred mass labels are substituted poly-aryl ethers. This application discloses a variety of ionisation methods and analysis by quadrupole mass analysers, TOF analysers and magnetic sector instruments as specific methods of analysing mass labels by mass spectrometry.

EP-A-0 711 362 (PCT/GB94/01675) disclose ligands, and specifically nucleic acids, cleavably linked to mass tag molecules. Preferred cleavable linkers are photo-cleavable. This application discloses Matrix Assisted Laser Desorption Ionisation (MALDI) Time of Flight (TOF) mass spectrometry as a specific method of analysing mass labels by mass spectrometry.

WO-A-98 26095 (PCT/US97/22639) discloses releasable non-volatile mass-label molecules. In preferred embodiments these labels comprise polymers, typically biopolymers which are cleavably attached to a reactive group or ligand, i.e. a probe. Preferred cleavable linkers appear to be chemically or enzymatically cleavable. This application discloses MALDI TOF mass spectrometry as a specific method of analysing mass labels by mass spectrometry.

WO-A-9727327 (PCT/US97/01070), WO-A-9727325 (PCT/US97/01046), WO-A-9727331 (PCT/US97/01304) disclose ligands, and specifically nucleic acids, cleavably linked to mass tag molecules. Preferred cleavable linkers appear to be chemically or photo-cleavable. These application discloses a variety of ionisation methods and analysis by quadrupole mass analysers, TOF analysers and magnetic sector instruments as specific methods of analysing mass labels by mass spectrometry.

The use of a 2-dansylethoxycarbonyl group as an intermediate 5'-OH protecting group in oligodeoxyribonucleotide synthesis is disclosed in Helvetica Chimica Acta, Vol 77, pages 203-215 (1994).

US 5,118,605 discloses methods for releasably binding a label to a support in the detection of a particular oligonucleotide sequence in a sample.

Bioconjugate Chem. 9,72-86(1998) discloses maleimidocysteineamido-DOTA derivatives as reagents for radiometal chelate conjugation to antibody sulphydryl groups.

There is still a need for improved linker groups having a number of properties:
- A good linker must permit the mass marker to be separated from its nucleic acid with high efficiency prior to detection within a mass spectrometer.
- Cleavage of the label from its nucleic acid should preferably be performed in-line with a mass spectrometer, possibly after some in-line pre-fractionation step such as capillary electrophoresis. This in-line cleavage step preferably does not require a complex interface with the mass spectrometer to enable this step to occur. Ideally linkers should cleave at some predetermined point within existing instruments without any modification to the instrument beyond changes of normal operating parameters.
- Linkers should cleave under mild conditions without causing cleavage or other reactions in the associated nucleic acids hence reducing noise in the mass spectrum from nucleic acid fragmentation and ionisation.
- Linkers should all cleave under the same conditions to ensure all labels can be analysed simultaneously and quantitatively.
- Linkers should be compatible with a wide variety of mass labels.
- Linkers should preferably be attachable to a nucleic acid in multiple positions.
- Linkers should be stable under conditions within conventional automated oligonucleotide synthesisers.

It is an object of this invention to provide linkers that have the desired features disclosed above which are compatible with existing mass spectrometers particularly electrospray ionisation mass spectrometry. It is thus an object of the present invention to provide compounds that allow easy attachment and detachment of reporter groups, defined below, to and from analytes, particularly proteins and nucleic acids.

Accordingly, the present invention provides a method for characterising an analyte, which method comprises:
(a) providing a compound in which the analyte is attached by a cleavable linker to a reporter group capable of identifying the analyte, wherein the reporter group comprises a mass marker detectable by mass spectrometry, the compound having the following formula: 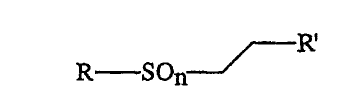 wherein either R comprises the reporter group and R' comprises the analyte, or R comprises the analyte and R' comprises the reporter group, and wherein n is 1 or 2;
(b) cleaving the reporter group from the analyte; and
(c) identifying the reporter group, thereby characterising the analyte.

The cleavable linker used in this invention is not especially limited, provided that it is a group linking R to R' as set out in the above formula. The linker may comprise substituents on the carbon atoms, provided that the substituents do not prevent the linker from being cleavable. The linker may be cleaved, for example, in the ion source of a mass spectrometer by loss of a proton followed by intra-molecular rearrangements resulting in the breaking of a covalent bond in the linker. This is essentially a thermal process but can be promoted, for instance, by increasing the cone voltage within the ion source of an electrospray mass spectrometer. Thus the linker is preferably a thermally cleavable linker or a linker cleavable by electron impact.

The analyte molecule is not particularly limited, and may be any molecule of interest. Typically in the present invention, the analyte molecule comprises a biological molecule. Preferred biological molecules include a protein, a polypeptide, an amino acid, a nucleic acid (e.g. an RNA, a DNA, a plasmid, or an oligonucleotide), a nucleic acid base, and an organic molecule such as a pharmaceutical agent or a drug.

In the context of the present invention, the term amino acid is intended to broadly denote the twenty natural L-isomers of the alpha-amino acids, glycine, alanine, valine, leucine, isoleucine, serine, threonine, tyrosine, lysine, arginine, histidine, methionine, cysteine, phenylalanine, tryptophan, asparagine, glutamine, aspartic acid, glutamic acid and proline. It is also intended to denote all naturally modified forms of these amino acids. In addition the term amino acid is intended to denote the D-isomers of these amino acids and non-natural alpha amino acids that may be used as analogues whether in isolation or incorporated into synthetic or biosynthetic peptides.

The term polypeptide is intended to denote all polymers of amino acids, including dimers, short oligo-peptides, long polypeptides, whether synthetic or natural, and native proteins from biological samples, which may or may not be post-translationally modified. The term nucleotide is intended to denote both naturally occurring nucleotides and analogues, in particular the 2', 3' dideoxynucleotide analogues. The term polynucleotide is intended to denote short oligonucleotides, long polynucleotides, whether synthetic or natural, and native nucleic acids from biological samples.

In the present invention, other analytes of interest may also include, for example, carbohydrates, lipids, drug molecules, natural products derived from living organisms and synthetic compounds from encoded chemical libraries.

In the context of the present invention, the term reporter group is intended to denote a moiety that is detectable by some conventional method of detection. The reporter group is preferably a mass marker, that is detectable by mass spectrometry. Appropriate reporters also include fluorophores, radiolabels, chemiluminescent labels, electron capture labels.

In the following description of the present invention, reference is made to 'intervening linker' or 'linker' groups which may be used to connect analytes or reporters to form the compounds of this invention. A variety of linkers is known in the art which may be introduced as further linkers between the thermally cleavable linkers of this invention and their covalently attached analyte or reporter molecules. Oligo- or poly-ethylene glycols or their derivatives are widely used as linkers (Maskos, U. & Southern, E.M. Nucleic Acids Research 20: 1679 -1684, 1992) and may be used as the further linkers in the present invention. Succinic acid based linkages are also widely used although these are less preferred as they are generally base labile and are thus incompatible with the base mediated deprotection steps used in a number of oligonucleotide synthesisers.

Propargylic alcohol is a bifunctional linker that provides a linkage that is stable under the conditions of oligonucleotide synthesis and is a preferred further linker for use with this invention. Similarly 6-aminohexanol is a useful bifunctional reagent to link appropriately funtionalised molecules and is also a preferred further linker.

A variety of cleavable linker groups is known in the art and may be used in conjunction with the compounds of this invention. Photocleavable linkers are well known in the art.

Ortho-nitrobenzyl groups are well known in the art as photocleavable linkers particularly 2-nitrobenzyl esters and 2-nitrobenzylamines, which cleave at the benzylamine bond. For a review on cleavable linkers see Lloyd-Williams *et al*., Tetrahedron 49: 11065-11133, 1993, which covers a variety of photocleavable and chemically cleavable linkers.

When the analyte is a polynucleotide, the compounds of this invention can be formed by attaching a reporter group via a linker group to an polynucleotide at a number of locations in the polynucleotide. For conventional solid phase synthesisers the 5' hydroxyl of a terminal ribose or deoxyribose is the most readily accessible. Other favoured positions for modification are the 5' position in the pyrimidine heterocycle and the 7' position and 8' position in the purine heterocycle. These would all be appropriate positions to attach the compounds of this invention.

The 2' position on the ribose or deoxyribose heterocycle is also accessible for attachment of a linker group. This position can be modified to a considerable degree, including derivitisation with linkers to mass labels. The phosphate groups are also available for reaction with the compounds of this invention.

The compounds used in this invention are stable under conditions that occur in conventional protein or DNA analysis and under the conditions in automated peptide or oligonucleotide synthesisers. The compounds provided are compatible with a variety of mass spectrometric analytical techniques and ionisation methods such as matrix assisted laser desorption ionisation (MALDI), electrospray ionisation (ESI), thermospray ionisation or fast atom bombardment ionisation (FAB). Preferred mass spectrometry analytical techniques for use with the present invention also include pyrolysis and gas chromatography/mass spectrometry (GC/MS). Cleavage of the reporter from its protein or nucleic acid can be performed in-line with a mass spectrometer in some embodiments, possibly after a pre-fractionation step such as capillary electrophoresis (CE) or high performance liquid chromatography (HPLC). The in-line cleavage step does not require a complex interface with a mass spectrometer to enable this step to occur.

The invention will now be described in further detail by way of example only, with reference to the accompanying drawings, in which:
Figure 1 shows a generic linker for use in the present invention;
Figure 2 shows a model linker used in the present invention, FT 27;
Figure 3 shows a model linker used in the present invention, FT 28;
Figure 4 depicts a schematic of the synthesis of FT27 and FT 28;
Figure 5 depicts a schematic of the synthesis of CMM267B, a linker for attachment to the 5' hydroxyl group of a nucleotide or oligonucleotide;
Figure 6 depicts a schematic for the synthesis of FT48 and FT49;

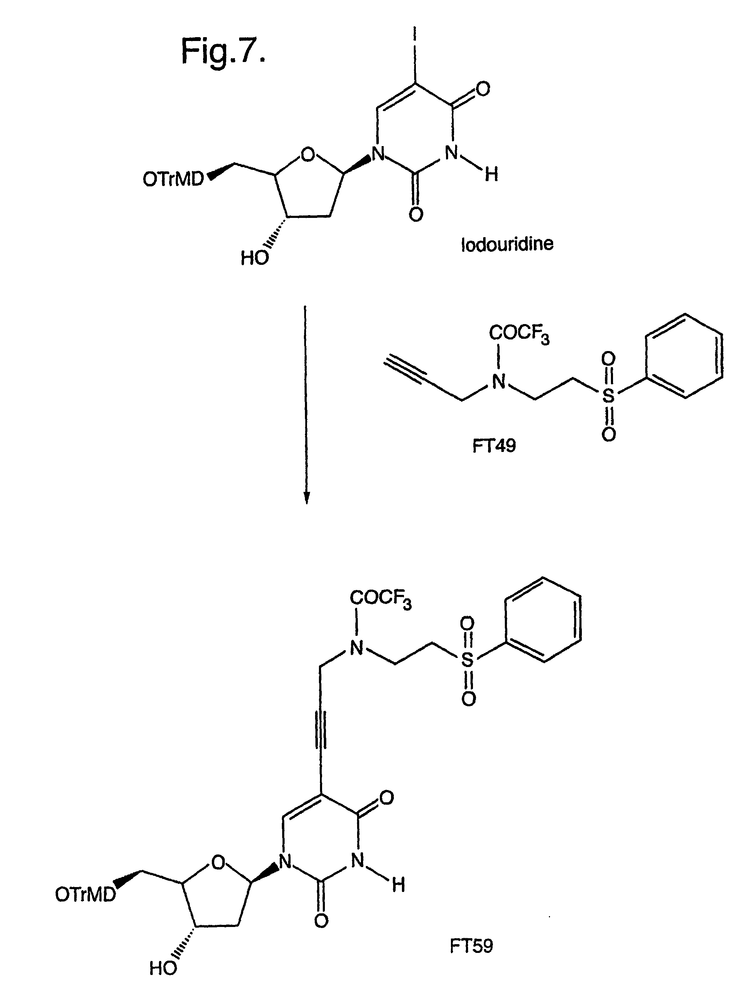

Figure 7 depicts a schematic for attachment of a linker (FT49) to a nucleobase to form FT59;
Figure 8 shows the negative ion mass spectrum of FT28;
Figure 9 shows the probable mechanism by which F28 cleaves;
Figure 10 shows a schematic diagram of the synthesis of a series of mass labels that are cleavable in an electrospray ion source;
Figure 11 shows the electrospray mass spectrum of FT134 which is an ether mass marker that is attached to a linker that is cleavable in an electrospray ion source;
Figure 12 shows the electrospray mass spectrum of FT135 which is an ether mass marker that is attached to a linker that is cleavable in an electrospray ion source;
Figure 13 shows the electrospray mass spectrum of FT136 which is an ether mass marker that is attached to a linker that is cleavable in an electrospray ion source;
Figure 14 shows the electrospray mass spectrum of FT137 which is an ether mass marker that is attached to a linker that is cleavable in an electrospray ion source;
Figure 15 shows the electrospray mass spectrum of FT142 which is an ether mass marker that is attached to a linker that is cleavable in an electrospray ion source;
Figure 16 shows the electrospray mass spectrum of FT143 which is an ether mass marker that is attached to a linker that is cleavable in an electrospray ion source;
Figure 17 shows the electrospray mass spectrum of FT146 which is an ether mass marker that is attached to a linker that is cleavable in an electrospray ion source; and
Figure 18 shows the electrospray mass spectrum of FT147 which is an ether mass marker that is attached to a linker that is cleavable in an electrospray ion source.

The present methods will now be described in more detail. As mentioned above, the present invention provides a method for characterising an analyte, which method comprises:
(a) providing a compound in which the analyte is attached by a thermally cleavable linker to a reporter group relatable to the analyte, the compound having the following formula: 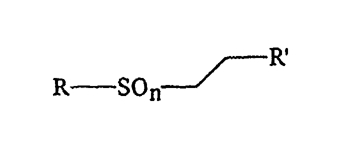 wherein either R comprises the reporter group and R' comprises the analyte, or R comprises the analyte and R' comprises the reporter group, and wherein n is 1 or 2;
(b) cleaving the reporter group from the analyte; and
(c) identifying the reporter group, thereby characterising the analyte.

The analyte and/or reporter group may be attached directly to the linker (e.g. R (or R') consists of the analyte or the reporter group), or through a further group (e.g. R (or R') consists of the analyte or the reporter group plus a further group. The reporter and/or analyte may be attached directly to the linker or further group, or may be attached through a covalent linkage, as described below. Preferably R and/or R' should be stable during synthesis of the reporter group, during incorporation of the reporter group into the analyte, e.g. into an oligonucleotide in an automated synthesiser. Preferably R and/or R' should also be stable during the detection of the reporter group, e.g. under mass spectrometry. A wide variety of groups have these properties and might be incorporated into the linker. It may also be desirable to choose substituents which change the solubility of the linker.

As mentioned above, preferably, R and/or R' comprise a covalent linkage formed in attaching the analyte and/or reporter group to the cleavable linker. The covalent linkage is not particularly limited provided that the reporter group and/or the analyte can readily be attached to the cleavable linker using reactive functionalities attached to the linker and the reporter and/or analyte. Typically, both R and R' comprise a covalent linkage, although in some embodiments only R or only R' comprises a covalent linkage.

Table 1 below lists some reactive functionalities that may be reacted together to generate a covalent linkage between two entities. Any of the functionalities listed below could be used to form the compounds used in the present invention to permit the linker to be attached to an analyte (such as a nucleic acid or protein) and to an appropriate reporter group for detection (e.g. by mass spectrometry). If desired, a reactive functionality can be used to introduce a further linking group with a further reactive functionality.

**Table 1**

| **Functionality 1** | **Functionality 2** | **Resultant Covalent Linkage** |
|---|---|---|
| -NH₂ | -COOH | -CO-NH- |
| -NH₂ | -NCO | -NH-CO-NH- |
| -NH₂ | -NCS | -NH-CS-NH- |
| -NH₂ | -CHO | -CH₂-NH- |
| -NH₂ | -SO₂Cl | -SO₂-NH- |
| -NH₂ | -CH=CH- | -NH-CH₂-CH₂- |
| -OH | -OP(NCH(CH₃)₂)₂ | -OP(=O)(O)O- |

It should be noted that some of the reactive functionalities above or their resultant covalent linkages might have to be protected prior to introduction into an oligonucleotide synthesiser. Preferably unprotected ether, ester, thioether and thioesters, amine and amide bonds are to be avoided as these are not stable in an oligonucleotide synthesiser. A wide variety of protective groups are known in the art to protect linkages from unwanted side reactions.

A short alkyl linkage would be appropriate to link the mass marker to the cleavable linker although a wide variety of linkers are available which can be used to link a mass marker to the tertiary amine group of the linker.

Thus, in preferred embodiments of the present invention, the covalent linkage attaching the cleavable linker to the reporter group and/or the analyte is independently selected from a -CO-NH- group, an -NH-CO-NH- group, an -NH-CS-NH- group, a -CH₂-NH- group, an -SO₂-NH- group, an -NH-CH₂-CH₂- group, or an -OP(=O)(O)O- group.

The R group in the compounds used in the present method is not particularly limited, provided that it comprises the reporter group or the analyte. The R group may thus comprise further groups, if desired. Typically R comprises, between the SOₙ group and the reporter group or analyte, a substituted or unsubstituted aromatic cyclic group, aliphatic cyclic group or heterocyclic group. Preferably R comprises a substituted or unsubstituted group selected from phenyl, pyridyl, pyranyl, naphthyl, anthracyl, pyrenyl, or fused ring derivatives or heteroaromatic analogues of the above. The substituents are not particularly limited and may comprise any organic group or a halogen (e.g. chlorine or bromine), preferably a hydrocarbon group, such as an alkyl group or a group comprising an alkene or alkyne functionality, a hydrocarbon group comprising a heteroatom, such as an N, O, P or S atom, or a cyclic group, such as an aromatic, aliphatic or heterocyclic group. The substituents may comprise a plurality of functional groups and may comprise straight chain or branched chain hydrocarbon groups.

When R comprises a phenyl group, the phenyl group is preferably a group having the following formula: 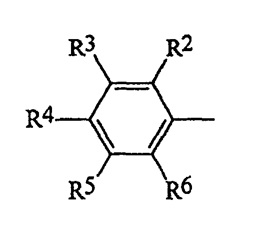 wherein one of R²-R⁶ comprises the reporter group or analyte, and the remaining R²-R⁶ groups are independently selected from a hydrogen, and a substituent as defined above, preferably a D, F, methyl, methoxy, hydroxy or amino group. It is particularly preferred that the R⁴ group comprises the reporter group or analyte.

The R' group in the compounds used in the present method is not particularly limited, provided that it comprises the reporter group or the analyte. The R' group may thus comprise further groups, if desired. Typically R' a group selected from -S-, -SO-, -NR¹-, and -O- between the SOₙ group and the reporter group or analyte. When the R' group comprises an -NR¹- group, preferably the R¹ group is an electron withdrawing group. More preferably, R¹ comprises a hydrogen atom, a halogen atom, or a substituent comprising a carbonyl group and/or a halogen atom. Thus, R¹ may comprise a fluorine atom, a chlorine atom, a bromine atom, an iodine atom, a trifluoroacetyl group, a trifluoromethyl acetate group, a mesylate group or a tosylate group.

In a particularly preferred embodiment of the present method, the compound provided in step (a) of the method has the following formula: 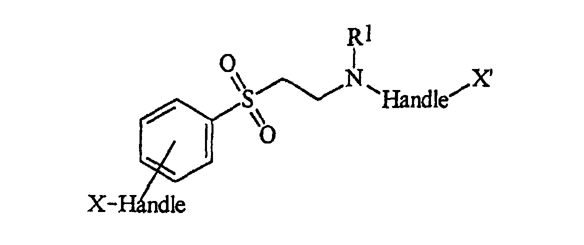 wherein R¹ is as defined above, X comprises the reporter group and X' comprises the analyte, or X comprises the analyte and X' comprises the reporter group, and each Handle is the same or different, being either a single bond directly attaching the X groups to the phenyl ring and the N atom respectively, or a covalent linkage as defined above.

In this embodiment of the present invention, the analyte is preferably attached to the linker through the phenyl group and the mass marker is preferably attached to the linker through the N atom. However, the analyte may also be attached to the linker through the N atom and the mass marker may also be attached to the linker through the phenyl group.

In this particular embodiment of the present invention, the analyte is preferably a nucleic acid or a nucleic acid base. Thus the analyte is preferably a DNA, an RNA an oligonucleotide or a plasmid. In this embodiment of the invention, the nucleic acid (or other analyte) is preferably attached to the linker through the phenyl group and the mass marker is preferably attached to the linker through the amine functionality.

In the method of the present invention, the analyte under investigation is not particularly limited and may be any molecule of interest. Typically the analyte comprises a biological molecule. In preferred embodiments of the present invention, the biological molecule is selected from a protein, a polypeptide, an amino acid, a nucleic acid (e.g. an RNA, a DNA, a plasmid, or an oligonucleotide), a nucleic acid base, a pharmaceutical agent or drug, a carbohydrate, a lipid, a natural product and a synthetic compound from an encoded chemical library. When the analyte comprises a nucleotide, oligonucleotide or nucleic acid, the nucleotide, oligonucleotide or nucleic acid may be natural, or may be modified by modifying a base, sugar and/or backbone of the nucleotide, oligonucleotide or nucleic acid.

Polypeptides from natural tissue samples often have cysteine residues in them which may be cross linked with other cysteine residues in the same polypeptide resulting in a disulphide bridge or cysteine linkage. These disulphide bridges may be broken, exposing free thiols, prior to labelling of the polypeptides. This step may be effected by reduction with an appropriate reagent such as beta-mercaptoethanol or dithiothreitol. When the analyte is an amino acid or a peptide comprising a cysteine group, the compound provided in step (a) of the present method preferably has the following formula: 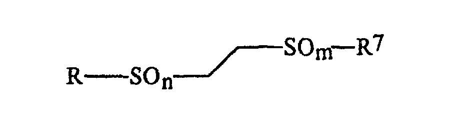 wherein R is as defined above and comprises a reporter group, m is 0 or 1 and the S atom attaching R⁷ to the linker is the sulphur atom of the cysteine group, R⁷ being the remainder of the amino acid or polypeptide. In the embodiment in which m=1, the sulphur of the cysteine has been oxidised. An appropriate reagent for this oxidation is, for example, hydrogen peroxide. This treatment renders the compound provided in step (a) more susceptible to thermal cleavage. This may be advantageous since certain types of analysis by mass spectrometry incorporate a step in which the compound is heated which would release the reporter group for analysis.

Alternatively, when the analyte is an amino acid or a peptide, the compound provided in step (a) may have the following formula: 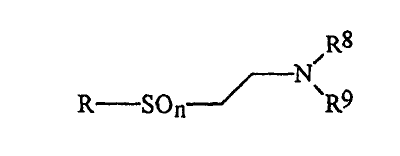 wherein R is as defined above and comprises a reporter group, the N atom is the nitrogen atom of an epsilon amino group of a lysine group, or is the nitrogen atom of an N-terminal alpha amino group, R⁸ is selected from H, O or an N-protective group, R⁹ being the remainder of the amino acid or polypeptide. Where one of -R⁸ or -R⁹ is oxygen, the compound is particularly thermally labile and will undergo a Cope elimination on heating. The amine oxide may, for example be generated by reaction of the amine with hydrogen peroxide.

In a further alternative, when the analyte is an amino acid or a peptide comprising a serine, threonine and/or tyrosine group, the compound provided in step (a) may have the following formula: 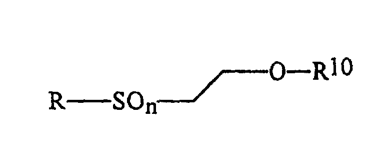 wherein R is as defined above and comprises a reporter group, the O atom is the oxygen atom from a hydroxyl group of the serine, threonine or tyrosine group, R¹⁰ being the remainder of the amino acid or polypeptide.

The reporter group used in the present invention is not especially limited and may be any group, provided that it is readily detectable and can se related to an analyte to identify the analyte. Typically, the reporter group is a mass marker, that is detectable by mass spectrometry. Other appropriate reporters include fluorophores, radiolabels, chemiluminescent labels, and electron capture labels.

In preferred embodiments of the present invention, mass markers disclosed in WO 98/31830, WO 99/32501 can be employed. WO 98/31830 and WO 99/32501 disclose poly-ether mass markers which are thermally stable, chemically inert and fragmentation resistant compounds, and which can be substituted with a variety of groups to alter properties such as solubility and charge. These mass markers are also preferred for use in the present invention. Markers which comprise two components, which may be poly-ethers, which are analysed by selected reaction monitoring are particularly preferred mass markers for use in the present invention. Mass markers that bind metal ions are also preferred markers for use with this invention. Reporter groups that can be detected by more than one detection means may also be desirable as with, for example, a fluorescent marker that incorporates a radioisotope in its linker and that is detectable by mass spectrometry and reporters of this kind are referred to as 'multi-mode reporter' groups. Preferred multi-mode reporter groups are detectable by mass spectrometry.

When the mass marker comprises an oligoether or a polyether, the oligoether or polyether may be a substituted or unsubstituted oligo- or poly-arylether. The oligoether or polyether preferably comprises one or more fluorine atom or methyl group substituents, or one or more ²H or ¹³C isotopic substituents.

It is further preferred that the mass marker comprises a metal ion-binding moiety. Typically, the metal ion-binding moiety comprises a porphyrin, a crown ether, hexahistidine, or a multidentate ligand. Preferably, the metal ion-binding moiety is a bidentate ligand or is EDTA. The metal ion-binding moiety may be bound to a monovalent, divalent or trivalent metal ion. The metal ion is not especially limited. Preferred metal ions include a transition metal ion, or a metal ion of group IA, IIA or IIIA of the periodic table. Particularly preferred metal ions are Ni²⁺, Li⁺, Na⁺, K⁺, Mg²⁺, Ca²⁺, Sr²⁺, Ba²⁺, or Al³⁺. The presence of a metal ion on the mass marker increases the sensitivity of detection.

It should be noted that this invention is not limited to the mass markers disclosed above. A number of features are desirable in a molecule that is to be a good mass marker. In particular, it is preferred that a marker is:
- be easily detachable from the analyte, such as DNA,
- not interfere with enzymatic processes involved in molecular biology protocols,
- be fragmentation resistant in a mass spectrometer,
- form a single ion peak in the mass spectrum,
- permit very sensitive detection,
- be easily distinguishable from background contamination, such as DNA; it should be possible to determine that a mass peak is from a mass label and not from contaminants,
- be compatible with conventional automated oligonucleotide synthesisers,
- be easy to synthesise in a combinatorial manner to minimise number of chemical steps and the number of reagents necessary to generate large number of labels, and
- be compatible with existing mass spectrometry instrumentation without requiring physical modification of the instruments.

As mentioned above, the linkers of the present invention are preferably thermally cleavable linkers. Thus, the present method preferably further comprises a step of heating the linker to cleave off the reporter group. The method of heating is not especially limited. Preferably the linker is heated within the device for detecting the reporter group, e.g. within a mass spectrometer. The linkers used in the present invention are advantageous in that they are thermally cleavable under mild conditions. It is for this reason that the mass markers can be cleaved from a molecule of interest within the mass spectrometer itself.

However, cleavage is not limited to thermal cleavage and may also include chemical cleavage. For example, in those embodiments of the present method in which the compound provided in step (a) has the following formula: 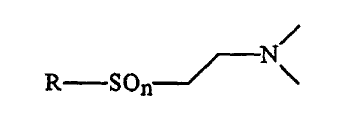 wherein n=1 or 2 and R is as defined above, the following chemical reaction steps may be employed to induce cleavage:
(i) reacting the above compound with an alkylating agent, to produce a quaternary ammonium derivative;
(ii) reacting the resulting quaternary ammonium derivative with a base, to release a compound of formula: 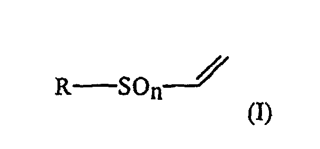 wherein R and n are as defined above; and
(iii) detecting this resulting compound by mass spectrometry.

Appropriate methylating agents include dimethyl sulphate and methyl iodide. This step can be carried out in a solvent, such as methanol. An appropriate base includes diisopropylethylamine, which may be dissolved in a further organic solvent, such as dichloromethane. This reaction is a Hoffman elimination and is well known in the art.

As mentioned above, the thermal cleavage preferably takes place in (or in-line with) a mass spectrometer. Thermal cleavage may take place within the inlet to a gas chromatography instrument or within the inlet to a gas chromatography/mass spectrometer, a pyrolysis mass spectrometer, a thermospray mass spectrometer or an electrospray mass spectrometer. The thermal cleavage may also evaporate the sample into the gas phase. In some embodiments thermal cleavage may be effected by laser, which may additionally desorb the reporter group for detection.

A second aspect of the present invention provides use of a linker group in the characterisation of an analyte, to attach a reporter group to the analyte, wherein the linker group is cleavable and has the following formula: 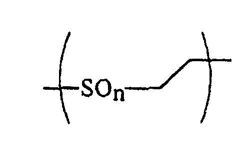 wherein n is 1 or 2.

The linker is a thus a cleavable linker, of the type described above in relation to the methods of the present invention. The analyte referred to in this aspect of the invention is not especially limited and may be any analyte as defined above. The reporter group referred to in this aspect of the invention is also not particularly limited and may be any reporter group as defined above.

The analyte and/or reporter group may be attached directly to the linker, or through a further group, such as the further groups described above. The reporter and/or analyte may be attached directly to the linker or further group, or may be attached through a covalent linkage, as described above.

In this aspect of the present invention; it is preferable that the linkers:
- cleave thermally under the conditions in an electrospray or thermospray ion source with a low cone voltage in an electrospray ion source, and/or
- are compatible with conventional oligonucleotide synthesis.

In a still further aspect, the present invention provides a compound having the following formula: 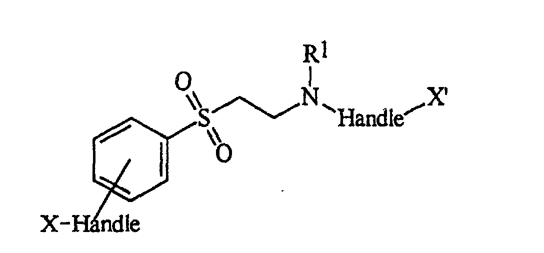 wherein R¹ is an electron withdrawing substituent, X comprises the reporter group and X' comprises the analyte, or X comprises the analyte and X' comprises the reporter group, and each Handle is the same or different, being either a single bond directly attaching the X groups to the phenyl ring and the N atom respectively, or a reactive group capable of attaching the X groups to the phenyl ring and the N atom respectively.

The compounds of this aspect of the invention are particularly preferred compounds of the type provided in step (a) of the method of the present invention. Thus, in the compounds of the present invention, n=2 and the group R defined above comprises a phenyl group substituted with a reporter group or analyte, attached to the phenyl group via a covalent linkage. Preferably the reporter group is attached to the phenyl group at a position *para* to the SO₂- group. The group R' defined above comprises an -NR¹- group attached via a covalent linkage to a reporter group or analyte.

In the compounds of the present invention the analyte is preferably attached to the linker through the phenyl group and the mass marker is preferably attached to the linker through the N atom. However, the analyte may also be attached to the linker through the N atom and the mass marker may also be attached to the linker through the phenyl group.

In this particular embodiment of the present invention, the analyte is not especially limited and is as defined above. Preferably the analyte is a nucleic acid or a nucleic acid base. Thus the analyte is preferably a DNA, an RNA an oligonucleotide or a plasmid. In this embodiment of the invention, the nucleic acid (or other analyte) is preferably attached to the linker through the phenyl group and the mass marker is preferably attached to the linker through the amine functionality.

In the compounds of the present invention, R¹ is as defined above, each Handle is as defined above and the reporter group is as defined above.

Preferred compounds of the present invention are essentially mass labelled oligonucleotides, and are useful in genetic analysis such as sequencing, polymerase chain reaction (PCR) based assays, and gene expression profiling. Arrays of mass labels will allow arrays of oligonucleotides to be labelled. These may be used as sequencing primers or primers for PCR or as probes for hybridisation assays, where in each case the analysis of multiple samples may be multiplexed.

### Formation of the compounds provided in step (a)

In a further aspect, the present invention provides a method for synthesising the compounds provided in step (a) of the present method.

The method of this aspect of the invention comprises reacting a compound of the following formula: 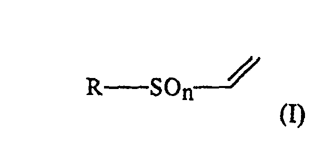 wherein R and n are as defined above;
with one or more reporter groups or analytes.

As mentioned above, when the analyte is an amino acid or a peptide comprising a cysteine group, the compound provided in step (a) of the present method preferably has the following formula: 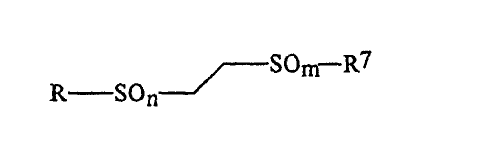 wherein R is as defined above and comprises a reporter group, m is 0 or 1 and the S atom attaching R⁷ to the linker is the sulphur atom of the cysteine group, R⁷ being the remainder of the amino acid or polypeptide. These compounds may be produced according to this aspect of the present invention by reacting compounds of formula (I) with free thiols on one or more amino acids or polypeptides, which may need to be reduced.

In this embodiment of this aspect of the invention, it is preferred that the reaction takes place for a relatively short period of time, preferably a few minutes. Any free thiols in an amino acid or polypeptide will react readily with compound (I). Some polypeptides may need to be reduced to expose free thiols as discussed above.

When the analyte is an amino acid or a peptide, the compound provided in step (a) may have the following formula: 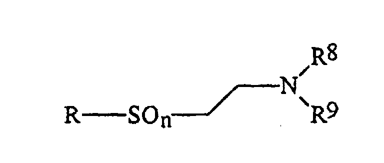 wherein R is as defined above and comprises a reporter group, the N atom is the nitrogen atom of an epsilon amino group of a lysine group, or is the nitrogen atom of an N-terminal alpha amino group, R⁸ is selected from H, O or an N-protective group, R⁹ being the remainder of the amino acid or polypeptide. These compounds may be produced according to this aspect of the present invention by reacting compounds of formula (I) with free amines on one or more amino acids or polypeptides.

In this embodiment of this aspect of the present invention, production of the amine-attached derivatives may be accompanied by simultaneous production of sulphur-attached derivatives (discussed above). In this embodiment of this aspect of the invention, it is preferred that the reaction takes place for a relatively long period of time as compared with the reaction forming the sulphur-attached derivatives. Free amines are less reactive than free thiols to compounds of formula (I). If an amino acid or polypeptide is reacted with compounds of formula (I), free thiols will react most rapidly, while free amines will react more slowly. The reaction times will depend to some extent on the precise nature of the compounds of formula (I) and conditions used, which may be determined empirically. To selectively react amines rather than thiols, the thiols may need to be blocked. This may be effected by reaction of free thiols with 4-vinylpyridine or iodoacetic acid. Disulphides may be broken and capped with performic acid. These techniques are well known in the art.

When the analyte is an amino acid or a peptide comprising a serine, threonine and/or tyrosine group, the compound provided in step (a) may have the following formula: 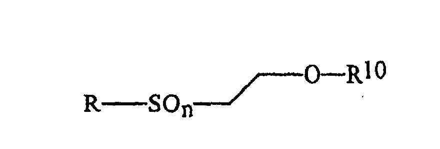 wherein R is as defined above and comprises a reporter group, the O atom is the oxygen atom from a hydroxyl group of the serine, threonine or tyrosine group, R¹⁰ being the remainder of the amino acid or polypeptide. These compounds may be produced according to this aspect of the present invention by reacting compounds of formula (I) with free hydroxyls on one or more amino acids or polypeptides.

In this embodiment of this aspect of the present invention, production of the oxygen-attached derivatives may be accompanied by simultaneous production of amine-attached and sulphur-attached derivatives (discussed above). In this embodiment of this aspect of the invention, it is preferred that the reaction takes place for an even longer period of time as compared with the reaction forming the sulphur-attached and amine attached derivatives. Free thiols and amines will react much faster with compounds of formula (I) than will free hydroxyls.

The method according to all of the embodiments of this aspect of the present invention may take place in an appropriate solvent. Appropriate solvents include acetonitrile or dimethylformamide. If a base is required, an appropriate base is triethylamine.

### Applications of the mass markers used in the invention

The mass markers employed in the present invention are not especially limited. Markers produced after cleavage of the cleavable linker have a wide range of structure. As already 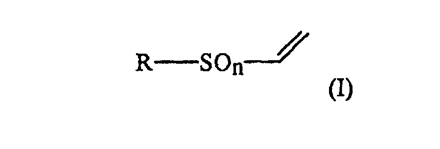 wherein R and n are as defined above.

These compounds are extremely useful reagents for analysis of amino acids, polypeptides, nucleotides, polynucleotides and other analytes of interest.

In particular, these compounds are useful in the analysis of amino acids and polypeptides by two dimensional (2-D) gel electrophoresis. 2-D gel electrophoresis is a technique for profiling protein expression, that is to say, cataloguing the identities and quantities of all the proteins expressed in a tissue (R.A. Van Bogelen., E.R. Olson, "Application of two-dimensional protein gels in biotechnology.", Biotechnol Annu Rev, 1:69-103, 1995). In this technique a protein mixture, extracted from a biological sample, is separated on a narrow gel strip. This first separation usually separates proteins on the basis of their iso-electric point. The entire gel strip is then laid against one edge of a rectangular polyacrylamide gel. The separated proteins in the strip are then electrophoretically separated in the second gel on the basis of their size by Sodium Dodecyl Sulphate Polacrylamide Gel Electrophoresis (SDS PAGE). Once the separation is complete the proteins are visualised. This typically involves staining the gel with a reagent that can be detected visually or by fluorescence. Radiolabelling and autoradiography are also used. In other methods fluorescent dyes may be covalently linked to proteins in a sample prior to separation. Covalent addition of a dye can alter the mobility of a protein and so this is sometimes less preferred, particularly if comparisons are to be made with public databases of 2-Dimensional gel images. Having visualised the proteins in a gel it is usually necessary to identify the proteins in particular spots on the gel. This is typically done by cutting the spots out of the gel and extracting the proteins from the gel matrix. The extracted proteins can then be identified by a variety of techniques. Preferred techniques involve digestion of the protein, followed by microsequencing or more preferably the digested proteins are analysed by MALDI TOF to generate a peptide mass fingerprint. (Jungblut P, Thiede B. "Protein identification from 2-D gels by MALDI mass spectrometry." Mass Spectrom Rev. 16:145-162, 1997).

This methodology is very difficult to automate and it is also relatively insensitive in its simplest incarnations. At present 2-D analysis is a relatively slow 'batch' process. It is also not very reproducible and it is expensive to analyse a gel. Since most of the costs in a gel based analysis are in the handling of each gel it would be desirable to be able to multiplex a number of samples on a 2-D gel simultaneously. In principle, if it were possible to label the proteins in different samples with a different, independently detectable tag, then the proteins in each sample could be analysed simultaneously on the same gel. This would be especially valuable for studies where it is desirable to follow the behaviour of the same proteins in a particular organism at multiple time points, for example in monitoring how a bacteria responds to a drug over a predetermined time course. Similarly comparing biopsy material from multiple patients with the same disease with corresponding controls would be desirable to ensure that the same protein from different samples would end up at the same spot on the gel. Running all the samples on the same gel would allow different samples to be compared without having to be concerned about the reproducibility of the separation of the gel. To achieve this requires a series of labels whose effect on the mobility of the proteins in different samples will be the same, so that a particular protein which is labelled with a different marker in each sample will still end up at the same position in the gel irrespective of its label.

It is anticipated that compounds of the formula (I), where R comprises mass markers which will alter the mobility of their associated proteins to the same extent, will be particularly effective for multiplexing 2-D PAGE analysis.

As mentioned above, the present invention also provides methods of forming compounds as defined in step (a) of the present methods by reacting a compound of the formula (I) with amino acids or polypeptides. Multiple samples can be labelled with different but related types of this compound that may be detected discretely by mass spectrometry. These labelled amino acids or polypeptides can then be separated by 2-D PAGE. In one method of analysing the resultant gel, the gel is electroblotted onto a target. The target is then raster scanned by laser so as to thermally cleave and desorb the reporter groups from their associated polypeptides for detection by mass spectrometry. In this method the whole gel or a region of the gel is completely imaged.

In an alternative embodiment, also employing compounds of formula (I), R may be a multi-mode reporter group, which comprises a mass marker and a radio-isotope is incorporated into the marker. Polypeptides labelled with compounds of this form on a 2-D gel could be detected by autoradiography which will provide an image of the gel indicating where protein is present. Small samples from the gel can then be cored out from regions of interest for analysis by pyrolysis mass spectrometry. Pyrolysis is advantageous, in that purification of the sample could be reduced or avoided. In preferred embodiments of the present invention, a radiolabel (e.g. ¹⁴C) is incorporated in the cleavable linker, such that upon cleavage, the radiolabel remains in the analyte.

### Examples

### Synthesis of a model linker compound (FT28) that cleaves with high efficiency in ESI sources

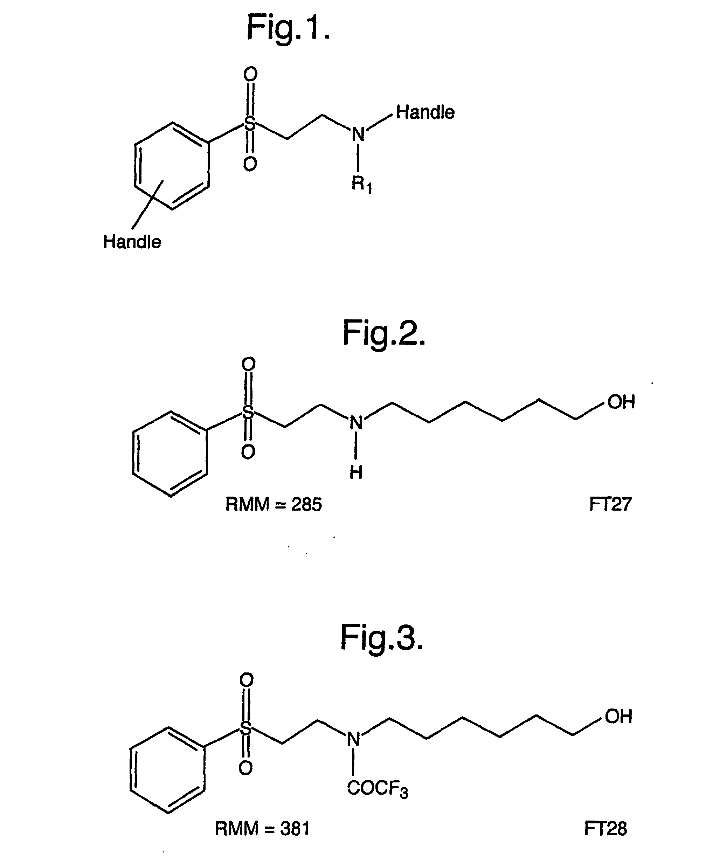

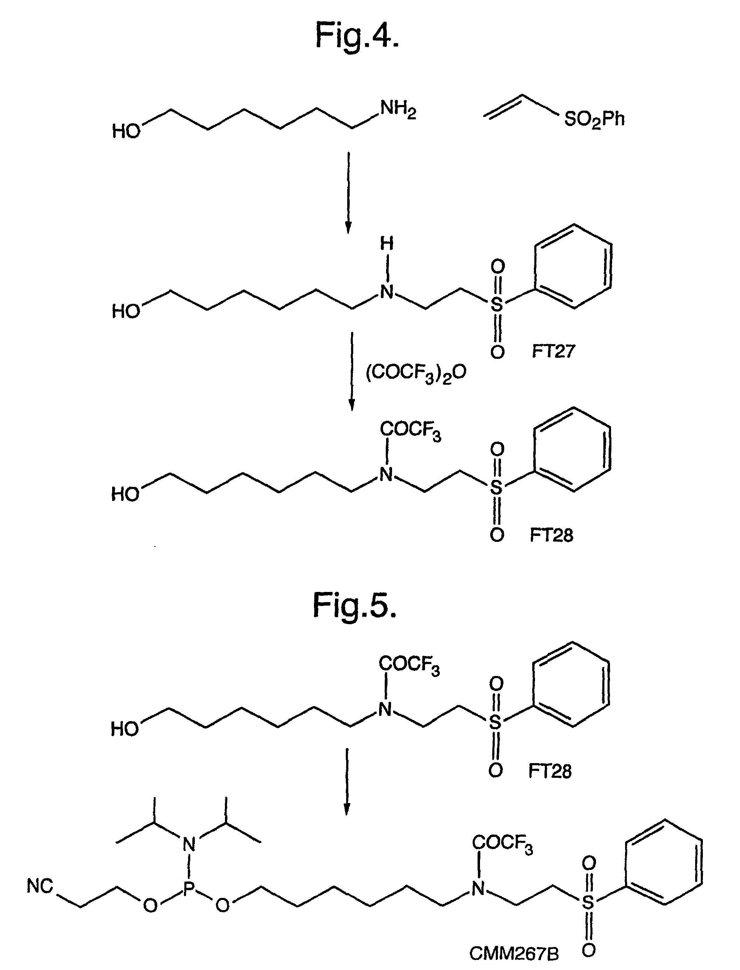

A model linker, shown in Figure 2 was synthesised (see Figure 4). This compound is identified as FT27 hereafter.

### Synthesis of FT27

A solution of phenyl vinyl sulphone (1.68 g, 10 mmol) and 6-aminohexanol (1.17 g, 10 mmol) in dry methanol (20 ml) was treated with triethyl amine (0.1 ml), refluxed for two hours, and stirred at room temperature overnight. The solvent was removed under reduced pressure. The oily residue was dissolved in ethyl acetate and the solvent was evaporated to give a crystalline residue. Recrystallisation from ethyl acetate/*n*-hexane yielded 2.67 g (94 %) FT 27.

### Confirmation of Identity of FT27 Product

M.p. 60-61°C;
Calculated Atomic Composition: C 58.92, H 8.12, N 4.91; Measured Composition: C 58.91, H 8.14, N 4.89.
¹H NMR (CDCl₃) 1.30-1.55 (m, 10 H), 2.57 (t, *J* = 7 Hz, 2 H), 3.02 (t, *J* = 7 Hz, 2 H), 3.29 (t, *J*= 7 Hz, 2 H), 3.63 (t, *J* = 7 Hz, 2 H), 7.55-7.71 (m, 3 H), 7.90-7.96 (m, 2 H);
¹³C NMR (CDCl₃) 25.44, 26.80, 29.69, 32,53, 43.03, 49.33, 56.02, 62.71, 128.01, 129.40, 133.84, 139.58.

### Synthesis of FT28

A derivative of FT27 was synthesised (see Figure 4), identified as FT28 and shown in Figure 3, in which the amine group of FT27 was protected with a trifluoroacetate group.

A solution of FT27 (855 mg, 3 mmol) in dry dichloromethane (15 ml) and triethylamine (2.5 ml, 18 mmol) was treated at 0°C with trifluoroacetic anhydride (0.85 ml, 6 mmol) and stirred for 10 min at this temperature and then for 40 min at room temperature. The reaction mixture was poured into a mixture of phosphate buffer pH 7.0 (40 ml) and methanol (80 ml) and stirred for 15 min. The organic solvents were removed under reduced pressure and the remaining aqueous residue was treated with ethyl acetate. The organic phase was separated and washed with aqueous solutions of 5 % sodium bicarbonate, 10 % citric acid and water. The organic phase was dried with sodium sulphate and the solvents were removed under reduced pressure. The residue was purified by flash chromatography on silica gel with ethyl acetate/*n*-hexane (3:2) as eluent to afford 1.07 g (94 %) of FT28 as a waxy solid.

### Confirmation of Identity of FT28 Product

¹H NMR (CDCl₃) 1.30-1.48 (m, 4H), 1.50-1.68 (m, 4 H), 3.30-3.48 (m, 4 H), 3.65 (m, 2 H), 3.75 (t, *J* = 7 Hz, 2 H), 7.56-7.73 (m, 3H), 7.90-7.94 (m, 2H).

### Synthesis of a linker for attachment to the 5'hydroxyl of a nucleotide or oligonucleotide

FT28 (0.306 g, 0.80 mmol) was co-evaporated three times with freshly distilled THF and then dissolved in THF (1.5 ml). The solution was stirred under argon and diisopropylethylamine (0.45 g, 0.6 ml, 3.5 mmol) was added. This was followed by the dropwise addition of 2-cyanoethyl-N,N-diisopropylchlorophosphoramidite (0.2 g, 0.2 ml, 0.85 mmol) and the reaction was stirred for one hour after which time Thin Layer Chromatography showed the reaction to be complete. The mixture was diluted (DCM), washed (KCl_{(aq)}), dried (Na₂SO₄) and then evaporated to dryness. The residue was dissolved in DCM (2 ml) then purified by precipitation into dry ice/acetone cooled hexane. The residue was dissolved in acetonitrile (1ml) and the solution was passed through a Gelman aerodisc (0.45 mm) then evaporated to dryness. The residual foam was co-evaporated several times with DCM before being dried in a vacuum desiccator over P₂O₅. This gave CMM267B (0.35 g, 75 %) as a white foam. Rf_{CMM267B} = 0.79. Rf_{FT28} = 0.43, DCM/EtOAc 1:1, U.V.

### Synthesis of a linker for attachment to a nucleobase (FT49)

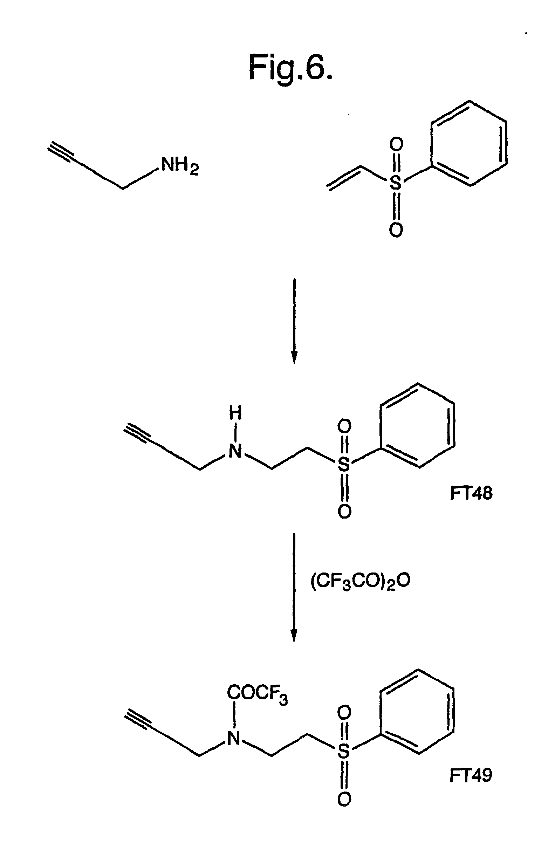

A model linker, shown in Figure 6 was synthesised via an intermediate identified as FT48. The model linker compound is identified as FT49 hereafter.

### Synthesis of FT48

A solution of phenyl vinyl sulphone (1.68 g, 10 mmol), propargylamine (605 mg, 11 mmol) and triethylamine (0.1 ml) in methanol (20 ml) was refluxed for 2.5 hours. The solvent was removed under reduced pressure to afford 2.23 g (100 %) FT48 which was used without further purification.

### Confirmation of Identity of FT48

¹H NMR (CDCl₃) 1.74 (br s, 1 H), 2.20 (t, *J* = 3 Hz, 1 H), 3.09 (t, *J* = 7 Hz, 2 H), 3.32 (t, *J* = 7 Hz, 2 H), 3.40 (d, *J* = 3Hz, 2 H), 7.55-7.71 (m, 3 H), 7.92-7.96 (m, 2 H).

### Synthesis of FT49

A solution of FT48 (2.0 g, 9 mmol) in dichloromethane (35 ml) was treated with triethylamine (2.5 ml), cooled to 0°C and subsequently treated with trifluoroacetic anhydride (2.3 g, 11 mmol). After stirring for 1 hour 40 min at this temperature the reaction was quenched with a saturated aqueous solution of ammonium chloride. The organic phase was separated, washed twice with water, dried with sodium sulphate, and evaporated to dryness under reduced pressure. The residue was purified by flash chromatoghraphy on silica gel with *n*-hexane/ethyl acetate (3:1) to furnish 2.77 g (96 %) FT49 as a colourless oil.

### Confirmation of Identity of FT48

¹H NMR (CDCl₃) 2.30-2,40 (m, 1 H), 3.50 (m, 2 H), 3.93 (m, 2 H), 4.28 (m, 2 H), 7.54-7.76 (m, 3 H), 7.85-7.96 (m, 2 H).

### Attachment of a linker to a nucleobase (FT59)

A solution of 5'-(4,4'-dimethoxytrityl)-5-iodouridine (656 mg, 1 mmol) in dry *N*,*N*-dimethylformamide (5 ml) was treated in sequence with a solution of FT49 (957 mg, 3 mmol) in dry *N*,*N*-dimethylformamide (5 ml), copper iodide (38 mg, 0.2 mmol), triethylamine (0.7 ml, 5 mmol), and palladium tetrakistriphenylphosphine (115 mg, 0.1 mmol). The reaction mixture was stirred for 6 hours at room temperature. The solvents were removed by co-evaporation with toluene under reduced pressure. The residue was dissolved in dichloromethane and washed with aqueous solutions of 5 % Na₂-EDTA, 10 % sodium thiosulphate, and water. The organic phase was dried with sodium sulphate and evaporated under reduced pressure. The residue was purified by flash chromatography on silica gel with dichloromethane/methanol (95:5) and subsequently with ethyl acetate/n-hexane/ethanol/triethylamine (65:30:5:1) to yield 650 mg (77 %) FT59 as a pale yellow foam.

### Confirmation of Identity of FT59

Calculated Atomic Composition: C 60.91, H 4.75, N 4.96; Measured Composition: C 60.73, H 4.74, N 4.93.
¹H NMR (CDCl₃) 2.31 (m, 1 H), 2.51 (m, 1 H), 3.31-3.66 (m, 6 H), 3.79 (s, 6 H), 4.06-4.15 (m, 3 H), 4.52 (m, 1 H), 6.31 (m, 1 H), 6.84 (d, *J* = 10 Hz, 4 H), 7.20-7.67 (m, 14 H), 7.87 (m, 2 H), 8.10-8.18 (2 s, 1 H);
MS (FAB) *m*/*z* 848 [M+H]⁺.

### Mass Spectrometry of model linkers FT27 and FT28

All data was acquired on a Platform-LC quadrupole instrument (Micromass Ltd, UK) with an electrospray ionisation source.

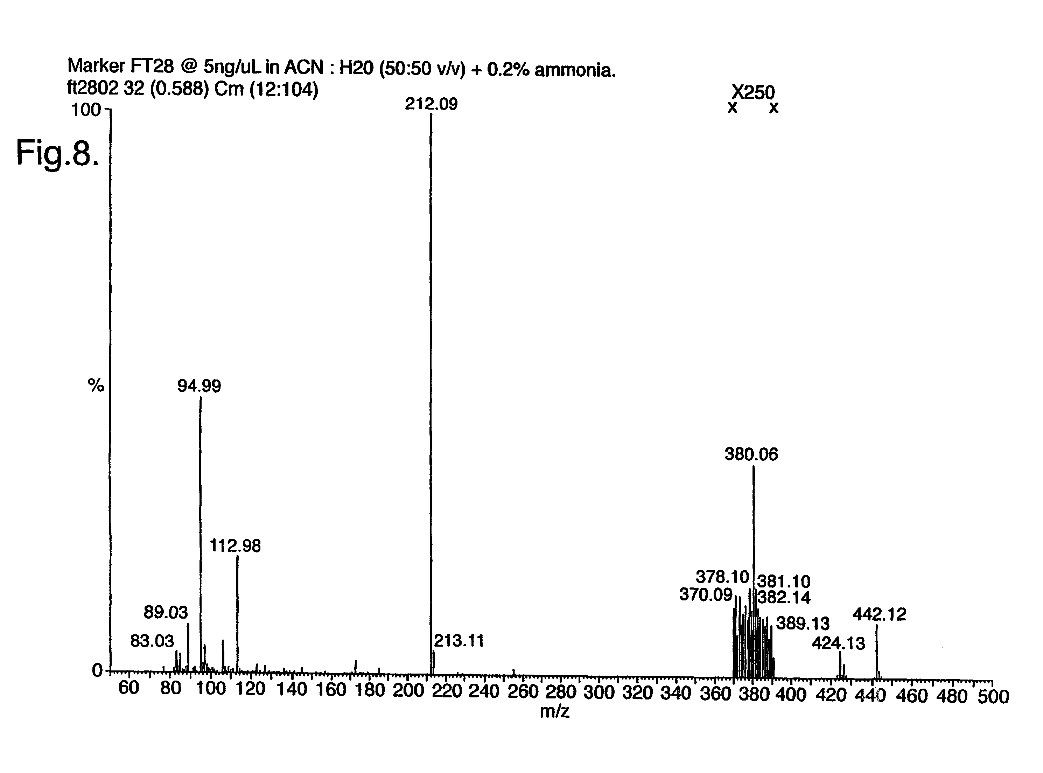

A solution of 5ng/ml of FT28 in a 50:50 mixture of water and acetonitrile was prepared. Ammonia was also present at 0.2 %. Figure 8 shows the negative ion mass spectrum of FT28. There is a relatively small peak at 380.1 corresponding to the molecular minus a single proton.

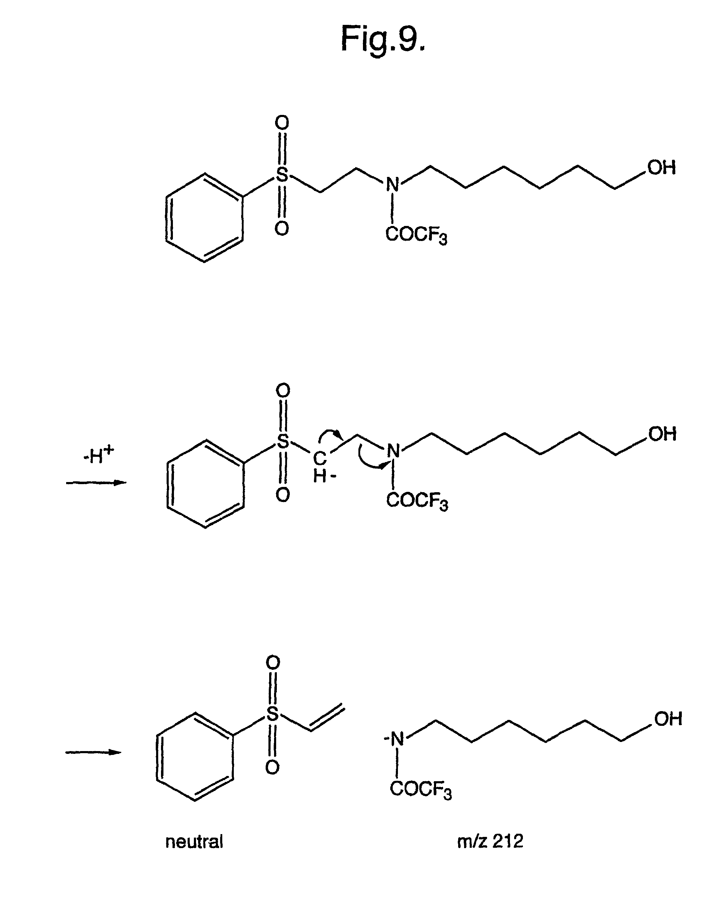

The region from 375-385 containing this peak has been magnified by 250x in Figure 8. There is a second much larger peak at 212.1 corresponding to the main cleavage product of FT28 that results from the ionisation of FT28. Figure 9 shows the probable mechanism by which the FT28 linker cleaves.

It should be noted that the trifluoroacetate group in FT28 protecting the amine linkage is unstable to strong base which is used in the final deprotection steps in an oligonucleotide synthesiser. This means that a different group must be found to replace this group which is stable to oligonucleotide synthesis to generate a linker that cleaves with high efficiency. Furthermore a reactive functionality must be substituted into the phenyl ring to provide the second handle required for this entity to be useful as a linker group.

### Synthesis of eight model mass labels that are cleavable in an electrospray ion source

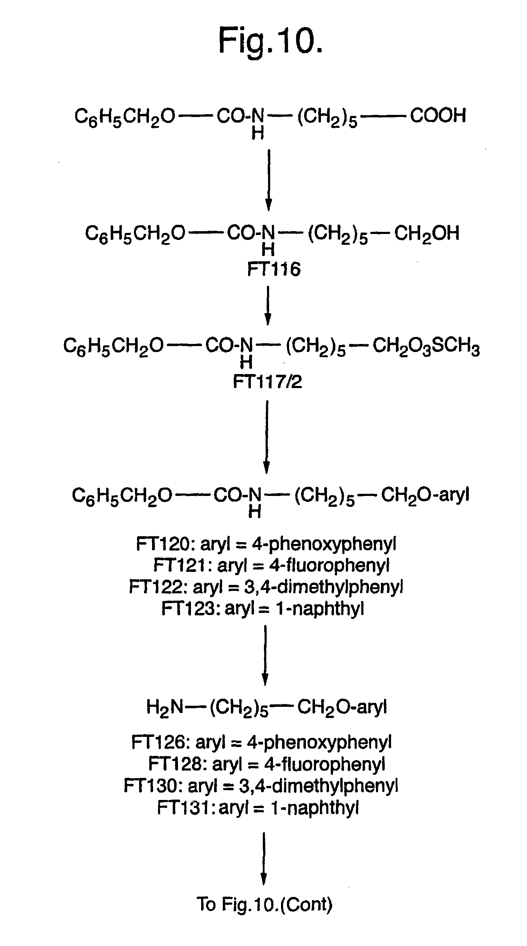

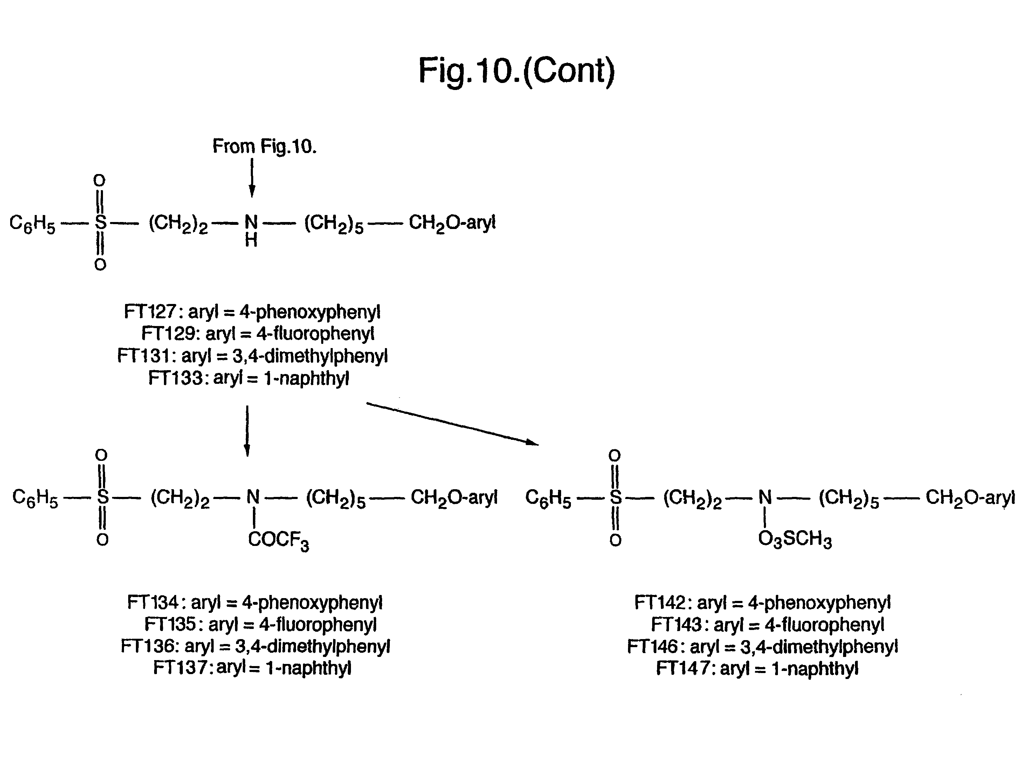

Eight ether compounds were synthesised from commercially available intermediates. These compounds were attached to linkers that cleave in an electrospray ion source. Two such linkers are used, one which contains a trifluoroacetate substitution protecting an amide group and one which contains a mesylate protective protecting the same amide group in the linker. Each of these linkers is attached to the same set of four ether mass labels to give a total of eight labels. Figure 10 shows a schematic of the syntheses performed. These mass labels were synthesised to demonstrate the principle of cleavage of labels in an electrospray ion source. The results show that the nature of the protective group is not essential to the functioning of the cleavable linker and that the nature of the mass marker does not interfere with the cleavage process. Thus this linker should be compatible with a wide variety of ether and polyether mass labels enabling the generation of large arrays of labels. The markers shown are model markers and are not attached to any analyte molecules. The core of the cleavable linker comprises a phenyl vinyl sulphone. The phenyl ring could be substituted with a bromine group, for example, to permit attachment to 1,7-octadiyne (Aldrich) which could then be used to react the marker group with 5'-(4,4'-Dimethoxy)trityl-5-iodouridine to generate nucleotide with a mass marker attached to a base. Similarly a brominated phenyl vinyl sulphone could be reacted with propargylic alcohol which may be O-protected (Aldrich) which would provide a free hydroxyl to attach the mass labels to other positions within a nucleotide using standard methods known in the art. A variety of other groups might be introduced to enable these labels to be reacted with other analytes such as proteins, carbohydrates or other biomolecules.

### Synthesis of FT116

A solution of *N*-benzyloxycarbonyl 6-aminocaproic acid (2.65 g, 10 mmol) in tetrahydrofuran (15 ml) was treated at 0°C with a 10 M solution of the borane dimethylsulphide complex in tetrahydrofuran (2.2 ml, 22 mmol) and stirred for 1 hour at 0°C and for 2 hours at room temperature. The reaction mixture was quenched by careful addition of methanol (2 ml). Subsequently the solvents were removed under reduced pressure and co-evaporated with methanol (3x20 ml) to give 2.416 g (96 %) of FT116. The crude product, which contains a non-polar impurity, was used without further purification in the next step.

An analytically pure sample was prepared by recrystallisation from ethyl acetate/*n*-hexane.

### Confirmation of Identity of FT116 Product

M.p. 79-81°C
Calculated Atomic Composition C 66.90, H 8.42, N 5.57; Measured Composition C 67.17, H 8.68, N 5.67.
¹H NMR (CDCl₃): 1-35-1.64(9 H, m), 3.19 (2 H,dt, *J*=6.5 and 6.5 Hz), 3.62(2 H, t, *J*= 6.5 Hz), 4.79 (1 H, br s), 5.10 (2 H, s), 7.26-7.37 (5 H, m).
¹³C NMR (CDCl₃): 25.31, 26.37, 29.96, 32.57, 40.95, 62.72, 66.64, 128.14, 128.57, 136.76, 156.57.

Mass spectrometry of the compound using Electron Impact Ionisation to ionise the compound gave peaks in the mass spectrum with the following mass to charge ratios: 251 (M⁺, <1 %), 160, 144, 130, 108, 91(100%).

### Synthesis of FT117/2

A solution of FT116 (2.3 g, 9.2 mmol) in dichloromethane (45 ml) and dry triethylamine (5 ml) was treated at 0-5°C with methanesulphonyl chloride (1.375g, 12 mmol). The reaction mixture was allowed to warm up to room temperature within 15 minutes and stirred for 4 hours at this temperature, diluted with dichloromethane (50 ml) and washed with a 5 % aqueous solution of sodium bicarbonate and water (2x). The organic phase was dried with sodium sulphate and the solvent was removed under reduced pressure. The residue was purified by flash chromatography on silica gel (100 g) using *n*-hexane/ethyl acetate (1:1) as eluent to furnish 2.413 g (80%) of FT117/2.

### Confirmation of Identity of FT117/2 Product

M.p. 24-27°C
Calculated Atomic Composition: C 54.69, H 7.04, N 4.25; Measured Composition: C 54.88, H 7.07, N 4.21.
¹H NMR (CDCl₃): 1.32-1.60 (6 H, m), 1.76(2H,m), 2.99 (3 H, s), 3.18(2H,m), 4.21(2H, t, *J*=6.5Hz),4.76(1H, br s), 5.10 (2 H, s), 7.26-7.35 (5 H, m).
¹³C NMR (CDCl₃): 25.09, 26.08, 29.03, 29.82, 37.42, 40.89, 66.66, 69.84, 128.16, 128.58, 136.75, 156.51.
Mass spectrometry of the compound using Electron Impact Ionisation to ionise the compound gave ions with the following mass to charge ratios: 329 (M⁺, 3 %), 222, 194, 126, 108, 91(100%).

### Synthesis of FT120, FT121, FT122, FT123 - General Procedure

A suspension of a 60% oily suspension of sodium hydride (120 mg, 3 mmol) in *N*,*N*-dimethylformamide (3 ml) was treated with a solution of the corresponding phenol (3 mmol) in *N*,*N*-dimethylformamide (2 ml). After finishing of the hydrogen evolution a solution of FT117/2 (493 mg, 1.5 mmol) *N*,*N*-dimethylformamide (2 ml) was added. The reaction mixture was stirred at room temperature for 18 hours and diluted with diethyl ether/*n*-hexane (1:1, 25 ml). The solution was washed with water (2 x), 1 M aqueous potassium hydroxide (3x10 ml) and water (2x). The organic phase was dried with sodium sulphate and evaporated to dryness. Flash chromatography on silica gel (20g) using *n*-hexane/ethyl acetate (3:1) as eluent to afford the corresponding phenolether.

### Confirmation of Identity of FT120 Product

FT120: yield 75 %; M.p. 79-81°C (diethyl ether/*n*-hexane)
Calculated Atomic Composition C 74,44, H 6.97, N 3.34; Measured Composition C 74.51, H 6.98, N 3.32.
¹H NMR (CDCl₃): 1.34-1.59 (6 H, m), 1.80(2 H, t, *J*= 6.5 Hz), 3.21(2 H, dt, *J*= 6.5 and 6.5 Hz), 3.93 (2 H, t, *J* = 6.5 Hz), 4.73 (1 H, br s), 5.10(2 H, s), 6.81-7.06(7 H, m), 7.17-7.37 (7 H, m).
¹³C NMR (CDCl₃): 25.76,26.48, 29.22, 29.54, 41.05,66.66, 68.33, 115.59, 117.68, 120.83, 122.46, 128.15, 128.58, 129.65, 136.77, 150.18, 155.50, 156.49, 158.65.
Mass spectrometry of the compound using Electron Impact Ionisation to ionise the compound gave ions with the following mass to charge ratios: 419 (M⁺, 5 %), 311, 186(100%), 91, 77.

### Confirmation of Identity of FT121 Product

FT121: yield 79 %, colourless oil
Calculated Atomic Composition H 69.54, H 7.00, N 4.06; Measured Composition C 69.34, H 7.04, N 3.99.
¹H NMR (CDCl₃): 1.37-1.5 (6 H, m), 1.76 (2 H, t, *J =* 6.5 Hz), 3.20 (2 H, dt, *J* = *6.5* and 6.5 Hz), 3.89 (2 H, t, *J* = 6.5 Hz), 4.73 (1 H, br s), 5.10 (2 H, s), 6.78-6.98 (4 H, m), 7.26-7.37 (5 H, m).
¹³C NMR (CDCl₃): 25.72,26.46,29.17, 29.94,41.03,66.65, 68.49,115.77, 115.62, 115.93, 128.15, 128.58, 129.65,136.77, 155.29,156.49, 158.85.
Mass spectrometry of the compound using Electron Impact Ionisation to ionise the compound gave ions with the following mass to charge ratios: 345 (M⁺, 5 %), 234, 202, 112, 91 (100 %).

### Confirmation of Identity of FT122 Product

FT122: yield 72 %; M.p. 49-51°C (diethyl ether/*n*-hexane)
Calculated Atomic Composition C 74.33, H 8.22, N 3.94; Measured Composition C 74.61, H 8.33, N 3.88.
¹H NMR (CDCl₃): 1.25-1.57 (6 H, m), 1.75 (2 H, t, *J* = 6.5Hz), 2.18 (3 H, s), 2.22 (3 H, s), 3.19(2 H, dt, *J* = 6.5 and 6.5Hz), 3.90(2 H, t, *J* = 6.5Hz), 4.73(1 H, br s), 5.10(2 H, s), 6.61-6.70 (2H,m), 7.01(1 H, d, *J* = 8.2 Hz), 7.25-7.36(5 H, m).
¹³C NMR (CDCl₃): 18.72, 19.77, 25.78, 26.49, 29.25, 29.95, 41.06, 66.63, 67.81, 111.52, 116.27, 128.13, 128.58, 130.33, 136.77, 137.92, 156.40, 157.33.
Mass spectrometry of the compound using Electron Impact Ionisation to ionise the compound gave ions with the following mass to charge ratios: 355 (M⁺,15 %), 264, 246, 212, 122, 121, 107,91 (100%).

### Confirmation of Identify of FT123 Product

FT123: yield 80 %; M.p. 42-44°C (diethyl ether/*n*-hexane)
Calculated Atomic Composition: C 76.36, H 7.21, N 3.71; Measured Composition: C 76.48, H 7.23, N 3.69.
¹H NMR (CDCl₃): 1.30-1.63 (6 H, m), 1.93 (2 H, t, *J* = 6.5 Hz), 3.20(2 H, dt, *J* = 6.5 and 6.5 Hz), 4,11(2 H, t, *J* = 6.5 Hz), 4.74(1 H, br s), 5.10(2 H, s), 6.78 (1 H, d, *J* = 7.3 Hz), 7.24-7.50 (9 H, m), 7.72 (1 H, m), 8.26 (1 H, m).
¹³C NMR (CDCl₃): 26.01,26.55, 29.23, 30.01,41.09, 66.65,67.99, 104.67, 120.11, 122.11, 125.15, 125.87, 125.96, 126.39, 127.51, 128.15, 128.58, 134.63, 136.97, 137.92, 154.94, 156.52.
Mass spectrometry of the compound using Electron Impact Ionisation to ionise the compound gave ions with the following mass to charge ratios: 377 (M⁺, 15 %), 269, 234, 165, 144, 127, 115,91 (100%).

### Synthesis of FT126, FT128, FT130, FT132 - General Procedure

A solution of the corresponding *N*-benzyloxycarbonyl-protected phenolether (1 mmol) in methanol (20 ml) was treated with ammonium formate (250 mg, 4 mmol) and 10 % Palladium on charcoal (80 mg) and stirred for 15-60 minutes at room temperature. The reaction mixture was filtered through a pad of Celite. The filtrate was concentrated to dryness under reduced pressure. The residue was dissolved in water/ethyl acetate and 1 M potassium hydroxide was added. The organic phase was washed with 1 M potassium hydroxide, water (2 x) and dried with sodium sulphate. The solvents were removed under reduced pressure. The corresponding crude amines were used without further purification in the next step.

### Synthesis of FT127, FT129, FT131, FT133 - General Procedure

A solution of the corresponding amine FT126, FT128, FT130 or FT132 (1 mmol) in methanol (10 ml) was treated with phenyl vinyl sulphone (168 mg, 1 mmol) and triethylamine (0.05 ml) and refluxed for 2 hours. The solvents were removed under reduced pressure and the residue purified by flash chromatography on silica gel (20 g) with ethyl acetate/triethylamine (100:1) as eluent to yield the corresponding secondary amine.

### Confirmation of Identity of FT127 Product

FT127: yield 82 %; colourless oil
Calculated Atomic Composition: C 68.84, H 6.89, N 3.09; Measured Composition: C 69.16, H 7.00, N 3.00.
¹H NMR (CDCl₃): 1.33-1.49 (6 H, m), 1.75 (2 H, dt, *J* = 16 and 7Hz), 2.58 (2 H, t, *J* = 7 Hz), 3.02(2H,t, *J*=7Hz), 3.29(2H,t, *J =* 7 Hz), 3.93(2H,t, *J*=7Hz), 6.85-7.06 (7 H, m), 7.26-7.32 (2 H, m), 7.55-7.69 (3 H, m), 7.91-7.94 (2 H, m).
Mass spectrometry of the compound using Electron Impact lonisation to ionise the compound gave ions with the following mass to charge ratios: 453 (M⁺, 3 %), 312,298, 285, 268, 198 (100%), 186, 168,141, 125, 100, 77.

### Confirmation of Identity of FT129 Product

FT129: yield 78 %; colourless oil
Calculated Atomic Composition: C 63.30, H 6.91, N 3.69; Measured Composition: C 63.50, H 6.96, N 3.65.
¹H NMR (CDCl₃): 1.33-1.49(6 H, m), 1.73(2 H, dt, *J* = 16 and 7 Hz), 2.57 (2 H, t, *J* = 7 Hz), 3.02(2 H, t, *J* = 7 Hz), 3.29 (2 H, t, *J* = 7 Hz), 3.90 (2 H, t, *J* = 7 Hz), 6.80-6.85 (2 H, m), 6.92-7.00 (2 H, m), 7.55-7.69 (3 H, m), 7.91-7.94 (2 H, m).
¹³C NMR (CDCl₃): 25.92, 26.96, 29.21, 29.87, 43.16, 49.46, 56.16, 68.56, 115.48, 115.61, 115.92, 128.01, 129.40, 133.83, 139.61, 155.33, 158.83.
Mass spectrometry of the compound using Electron Impact Ionisation to ionise the compound gave ions with the following mass to charge ratios: 380 [M+1]⁺ (1 %), 378 [M-1]⁺ (1 %), 287, 268, 198 (100%), 141, 125, 126, 112.

### Confirmation of Identity of FT131 Product

FT131: yield 93 %; colourless oil
Calculated Atomic Composition: C 67.83, H 8.02, N 3.60; Measured Composition: C 67.89, H 8.06, N 3.55.
¹H NMR(CDCl₃): 1.31-1.49 (6H, m), 1.75(2H,dt, *J* = 16 and 7 Hz), 2.18 (3 H, s), 2.22 (3 H,s), 2.56 (2 H, t, *J* = 7 Hz), 3.01 (2H, t, *J* = 7 Hz), 3.29 (2H, t, *J =* 7 Hz), 3.91 (2 H, t, *J* = 7 Hz), 6.62 (1 H, dd, *J* = 9 and 2.5 Hz), 6.70 (1 d, *J =* 2.5 Hz), 7.01 (1 H, d, *J*= 9 Hz), 7.55-7.69 (3 H, m), 7.90-7.94 (2 H, m).
¹³C NMR (CDCl₃): 18.72, 19.97, 25.97, 26.98, 29.29, 29.89, 43.17, 49.48, 56.17, 67.87, 111.52, 116.27,128.02, 128.48, 129.41, 130.32, 133.83, 137,61, 139.61, 157.35,
Mass spectrometry of the compound using Electron Impact Ionisation to ionise the compound gave ions with the following mass to charge ratios: 389 (M⁺, 2 %), 268, 198 (100 %), 141, 126, 122, 107.

### Confirmation of Identity of FT133 Product

FT133: yield 85 %, colourless oil
Calculated Atomic Composition: C 70.04, H 7.10, N 3.40; Measured Composition: C 69.80, H 7.13, N 3.38.
¹H NMR (CDCl₃): 1.35-1.62 (6 H, m), 1.92 (2 H, dt, *J=* 16 and 7 Hz), 2.56 (2 H, t, *J* = 7 Hz), 3.02 (2 H, t, *J* = 7 Hz), 3.28 (2 H, t, *J =* 7 Hz), 4.13 (2 H, t, *J* = 7 Hz), 6.89 (1 H,dd, *J* = 8 and 1.3 Hz), 7.33-7.68 (7 H, m), 7.78 (1 H, m), 7.90-7.94 (2 H, m), 8.27 (1 H, m).
¹³C NMR (CDCl₃): 26.18, 27.00, 29.23, 29.92,43.18,49.48, 56.17, 68.02, 104.67, 120.07, 122.11, 125.11, 125.97, 126.37, 127.49, 128.02, 129.39, 133.81, 134,62, 139.61, 154.96.
Mass spectrometry of the compound using Electron Impact Ionisation to ionise the compound gave ions with the following mass to charge ratios: 411 (M⁺, 5 %), 268, 198 (100 %), 144, 126, 125, 115, 100.

### Synthesis of FT134, FT135, FT136, FT137 - General Procedure

A solution of the corresponding secondary amine FT127, FT129, FT131 or FT133 (0.12 mmol) in dry dichloromethane (2 ml) was treated with triethylamine (0.25 ml) and finally at 0°C with trifluoroacetic anhydride (0.05 ml, 0.37 mmol). The reaction mixture was stirred at this temperature for 20 min and treated with methanol (0.25 ml). The solvents were evaporated under reduced pressure and the residue was dissolved in dichloromethane and washed with water. The organic extract was dried with sodium sulphate, evaporated to dryness and purified by flash chromatography on silica gel with *n*-hexane/ethyl acetate (2:1) to furnish the corresponding trifluoroacetamides FT134, FT135, FT136, and FT137.

### Confirmation of Identity of FT134 Product

FT134: yield 93 %, amorphous solid.
¹H NMR (CDCl₃): 1.34-1.84 (8 H,m), 3.30-3.48 (4 H, m), 3.76 (2 H, m), 3.95 (2 H; m), 6.84-7.10(7 H, m), 7.27-7.33 (2 H, m), 7.55-7.73 (3 H, m), 7.90-7.95 (2 H, m).
Mass spectrometry of the compound using Chemical Ionisation with Ammonia in the source to ionise the compound gave ions with the following mass to charge ratios: 567 [M+NH₄]⁺ (100 %), 549 [M]⁺, 475, 427, 399, 381, 186.

### Confirmation of Identity of FT135 Product

FT135: yield 82%; amorphous solid.
¹H NMR (CDCl₃): 1.34-1.82 (8 H, m), 3.30-3.48 (4 H, m), 3.76 (2 H, m), 3.90 (2 H, m), 6.79-6.85 (2 H, m), 6.92-7.00 (2 H, m), 7.55-7.73 (3 H, m), 7.90-7.95 (2 H, m).
MS (CI, NH₃): 493 [M+NH₄]⁺ (100%), 475 [M]⁺,381, 353, 351, 325, 186.

### Confirmation of Identity of FT136 Product

FT136: yield 98 %; amorphous solid.
¹HNMR (CDCl₃): 1.30-1.81 (8 H, m), 2.19 (3 H, s), 2.23 (3 H, s), 3.30-3.48 (4 H, m), 3.77 (2 H, m), 3.92 (2 H, m), 6.64 (1 H, dd,*J*= 9.5 and 2.5 Hz), 6.71 (1 H, d, *J* = 2.5 Hz), 7.02 (1 H, d, *J* = 9.5 Hz) 7.55-7.73 (3 H, m), 7.90-7.95 (2 H, m).
Mass spectrometry of the compound using Chemical Ionisation with Ammonia in the source to ionise the compound gave ions with the following mass to charge ratios: 503 [M+NH₄]⁺, 485 [M]⁺, 383, 363, 335, 318, 241, 186, 122,43 (100 %).

### Confirmation of Identity of FT137 Product

FT137: yield 80 %; amorphous solid.
¹H NMR (CDCl₃): 1.34-1.49 (2 H, m), 1.55-1.72 (4 H, m), 1.87-1.99 (2 H, m), 3.30-3.48 (4 H, m), 3.72 (2 H, m), 4.15 (2 H, m), 6.80 (1 H, dd, *J* = 8.5 and 1.5 Hz), 7.33-7.70 (7 H, m), 7.80 (1 H, m), 7.89-7.94 (2 H, m), 8.27 (1 H, m).
Mass spectrometry of the compound using Chemical Ionisation with Ammonia in the source to ionise the compound gave ions with the following mass to charge ratios: 525 [(M+NH₄)⁺, 100 %], [508 (M+1)⁺], 385, 383, 357, 340, 186, 160, 140.

### Synthesis of FT142, FT143, FT146, FT147- General Procedure

A solution of the corresponding secondary amine FT127, FT129, FT131 or FT133 (0.06 mmol) in dry dichloromethane (2 ml) was treated with triethylamine (0.1 ml) and finally at 0°C with methanesulphonyl chloride (0.1 ml, 0.13 mmol). The reaction mixture was stirred at this temperature for 20 min and treated with methanol (0.1 ml). The reaction mixture was diluted with dichloromethane and washed with water. The organic extract was dried with sodium sulphate, evaporated to dryness and purified by flash chromatography on silica gel with *n*-hexane/ethyl acetate (2:3) to furnish the corresponding sulphonamides FT142, FT143, FT146, and FT147.

### Confirmation of Identity of FT142 Product

FT142: yield 86%, amorphous solid.
¹H NMR (CDCl₃) 1.34-1.83 (8 H, m), 2.83 (3 H, s), 3.20 (2 H, t, *J* = 9 Hz), 3.41-3.47 (2 H,m), 3.57-3.62 (2 H, m), 3.94 (2 H, t, *J* = 7 Hz), 6.84-7.07 (7 H,m), 7.26-7.33 (2 H, m), 7.57-7.70 (3 H, m), 7.91-7.95 (2 H, m).
MS (DCI, NH₃): 549 [M+NH₄]⁺ 100 %, 531 M⁺, 409, 381, 223, 186.

### Confirmation of Identity of FT143 Product

FT143: yield 88 %; amorphous solid.
¹H NMR (CDCl₃) 1.30-1.65 (6 H, m), 1.71-1.81 (2 H, m), 2.83 (3 H, s), 3.19 (2 H, t, *J*= 9Hz), 3.41-3.47 (2 H, m), 3.56-3.62 (2 H, m), 3.91(2 H, t, *J* = 7Hz), 6.79-7.01 (4 H, m), 7.57-7.72 (3 H, m), 7.91-7.95 (2 H, m).
Mass spectrometry of the compound using Chemical Ionisation to ionise the compound gave ions with the following mass to charge ratios: 475 [M+NH₄]⁺, 458 [M+H]⁺, 363,307,238, 186.

### Confirmation of Identity of FT146 Product

FT146: yield 89 %; amorphous solid.
¹H NMR (CDCl₃) 1.32-1.63 (6 H, m), 1.70-1.80 (2 H, m), 2.19 (3 H, s), 2.23 (3 H, s), 2.82 (3 H, s), 3.18 (2 H, t, *J*= 9 Hz), 3.41-3.47 (2 H, m), 3.55-3.62 (2 H, m), 3.92 (2 H, t, *J*= 7 Hz), 6.63 (1 H, dd, *J* = 9.5 and 3 Hz), 6.71 (1 H, d, *J* = 3 Hz), 7.02 (1 H, d,*J*= 9.5 Hz), 7.56-7.72 (3 H, m), 7.91-7.95 (2 H, m).
MS (DCI, NH₃): 485 [M+NH₄]⁺(100 %), 467 [M]⁺, 363, 345,317, 248, 186, 122.

### Confirmation of Identity of FT147 Product

FT147: yield 99 %; amorphous solid.
¹H NMR (CDCl₃) 1.35-1.47 (2 H, m), 1.54-1.67 (4 H, m), 1.88-1.98 (2 H, m), 2.81 (3 H, s), 3.20 (2 H, t, *J* = 9 Hz), 3.41-3.47 (2 H, m), 3.55-3.62 (2 H, m), 4.14 (2 H, t, *J* = 7 Hz), 6.80 (1 H, d, *J*= 8.5 Hz), 7.32-7.51 (4 H, m), 7.54-7.7 (3 H, m), 7.80 (1 H, m), 7.91-7.95 (2 H, d, *J* = 8 Hz), 8.27 (1 H, m).
MS (DCI, NH₃): 507 [M+NH₄]⁺, 490 [M+1]⁺, 365, 339, 322, 206, 186 (100 %).

### ESI-MS Analysis of FT134, 135, 136, 137 and FT 142, 143, 146, 147

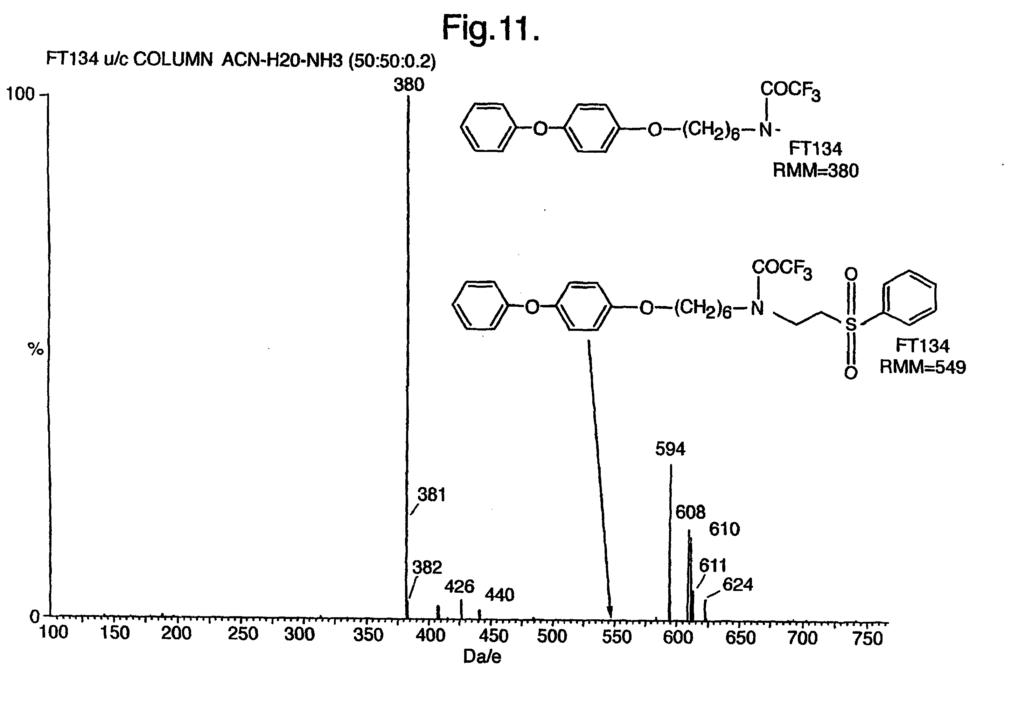

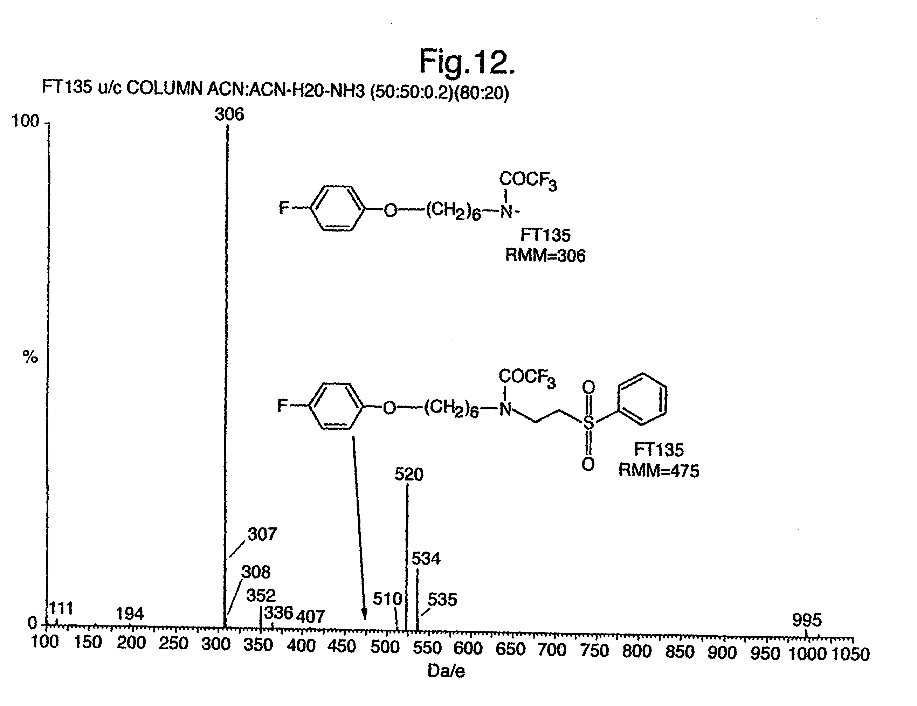

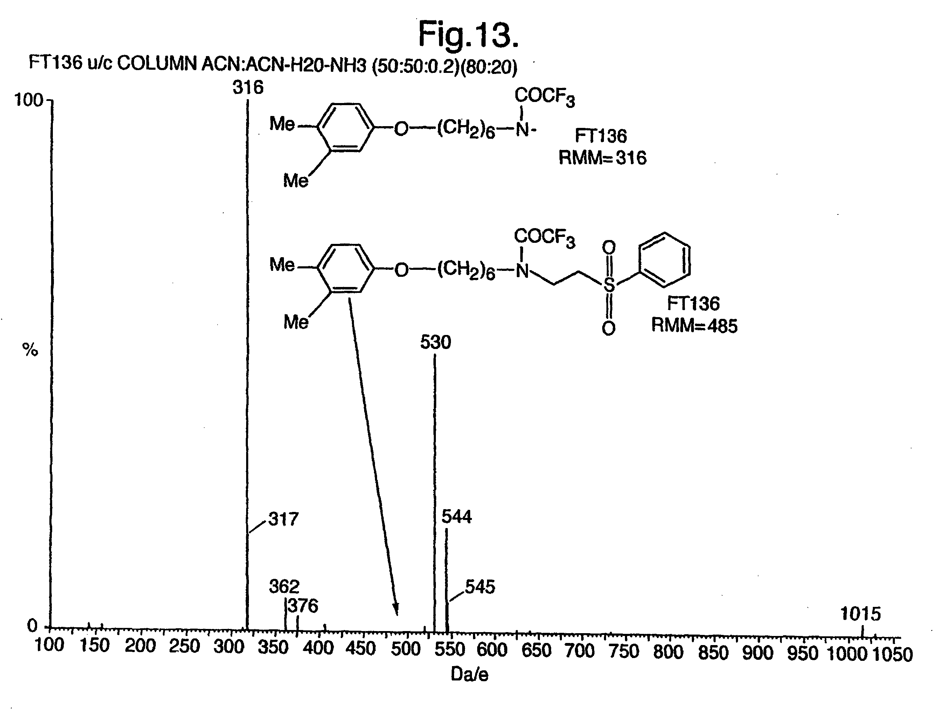

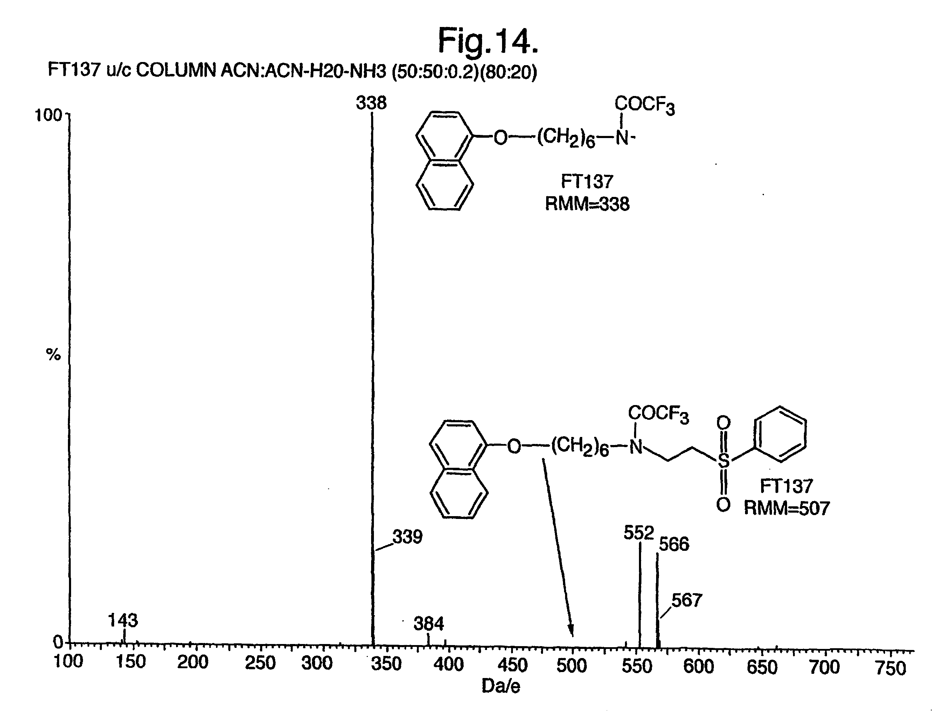

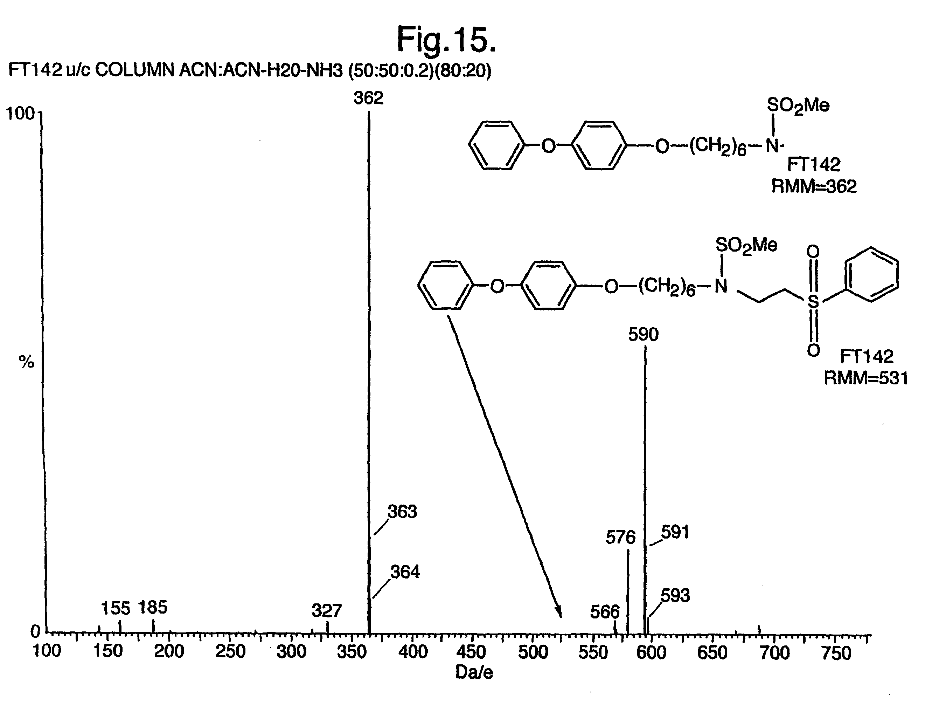

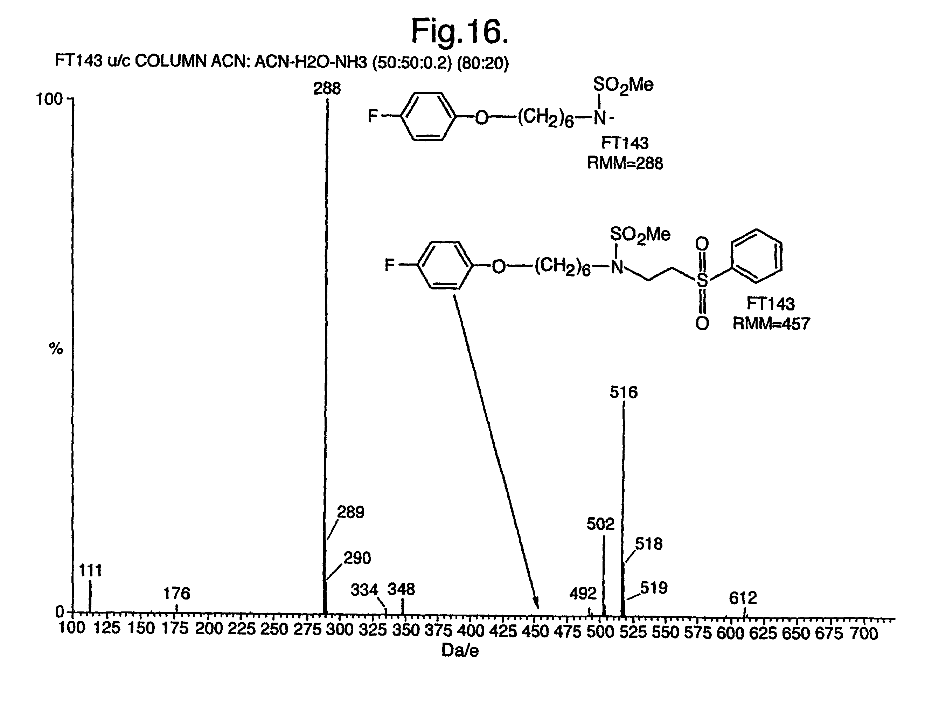

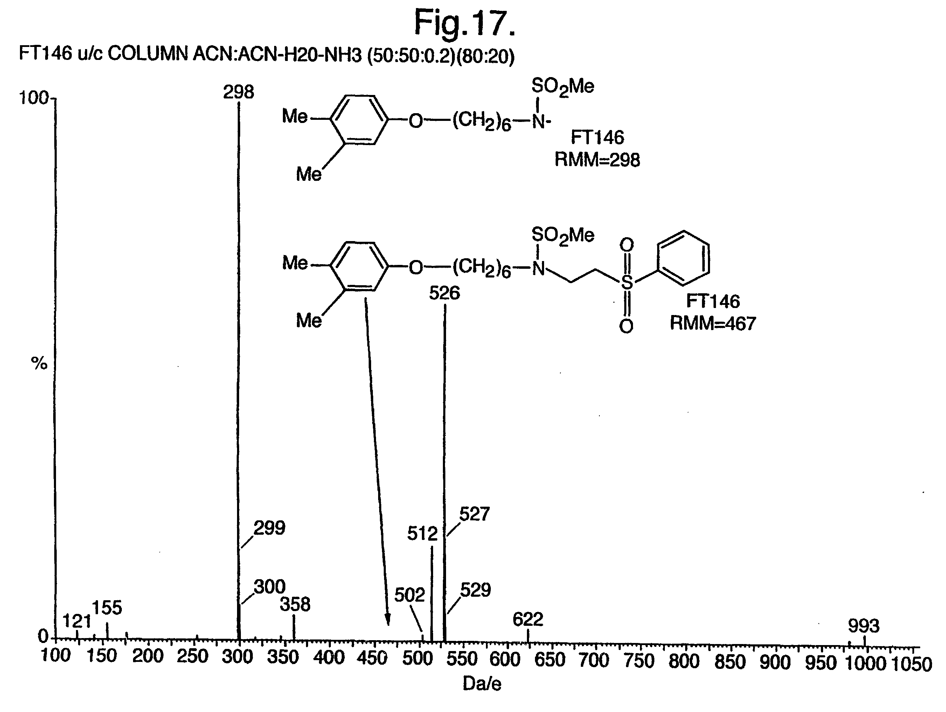

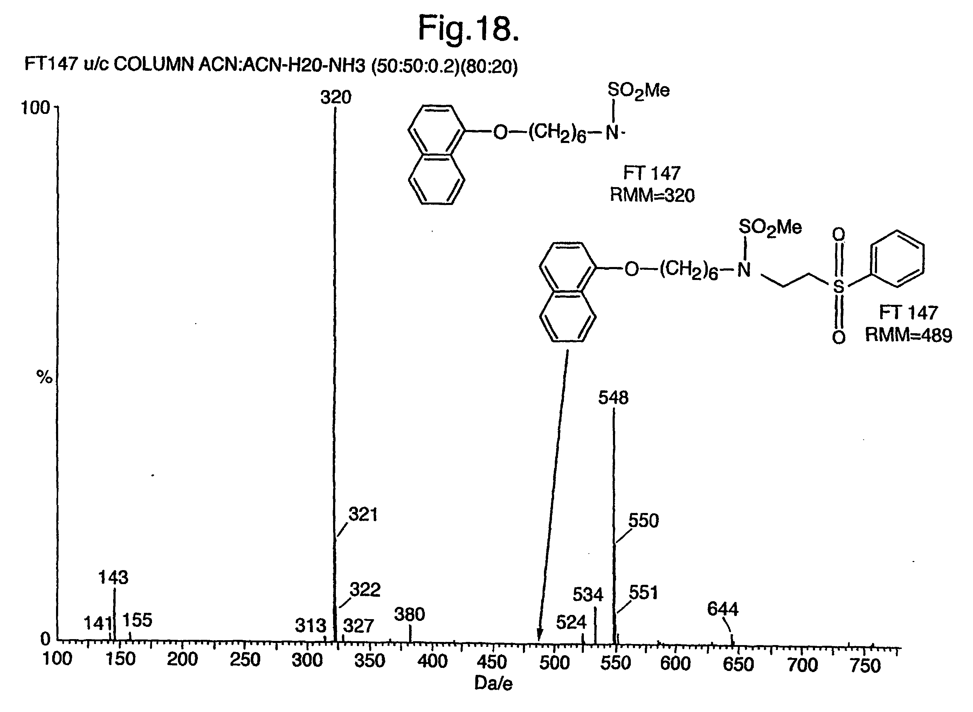

The eight compounds FT 134,135,136, 137 and FT 142, 143, 146,147 were analysed on a Platform-LC quadrupole instrument (Micromass Ltd, UK) with an electrospray ionisation source. Solutions of each the markers were made up in a 50:50 mixture of water and acetonitrile. Ammonia was also present in the solvent at a concentration 0.2 %. Figures 11 to 18 show the negative ion mass spectra of FT 134,135,136,137 and FT 142, 143, 146, 147 respectively. In each case there is no detectable molecular ion and a dominant peak in the spectrum corresponding to the negative ion cleavage product is identified in each case. In all of these spectra there are a number of additional peaks. The most significant of these occur at masses corresponding to the molecular mass of the uncleaved marker plus 45 daltons and plus 59 daltons which correspond to adducts of formate and acetate with the uncharged, uncleaved mass marker respectively. These were contaminants in the ion source of the mass spectrometer from previous use.

### Addition of the amino groups of the protein albumin to the vinyl double bond of phenyl-vinylsulphone

In a mixture of an organic solvent such as acetonitrile and water, the solubility of albumin is increased by the addition of neutral salts. 7 mg of the protein were dissolved in 1 ml of a solvent mixture Acetonitrile/Water (75 % Acetonitrile (ACN)). To the cloudy solution were added 2 drops of NaCl 0.1 molar solution and 2 drops of triethylamine (TEA). The solution became clear after addition of the salt. To the clear solution of albumin was added 6 mg of phenyl-vinylsulphone (PVS) in 1 ml of ACN. The reaction mixture was stirred at room temperature overnight. The solvents were evaporated under reduced pressure. The solid residue was then dissolved in a small amount of methanol and the very small amount of salt that precipitated out was removed by filtration. The filtrate was reduced by evaporation and the final product was crystallised from ether to give 6.5 mg of a white solid. The solid was washed several times with distilled ethyl acetate to remove any excess PVS. The last washing was retained for analysis and the solid was dried under reduced pressure.

The GC-MS instrument was first calibrated and adjusted to perform the detection of the 125 m/z ratio peak that is characteristic for the ArSO⁺ fragment from the parent molecular ion 168 m/z of PVS (CH=CH₂SO₂Ar). Neither blank solvent (pure ethyl acetate) nor the ethyl acetate from the last washing of PVS labelled albumin showed a 125 m/z peak. This means that the modified protein is not contaminated with any traces of free PVS.

### Cleavage of the phenyl-vinylsulphone from the modified Protein

5 mg of the adduct (albumin-phenyl-vinylsulphone) in 2 ml MeOH was reacted with two drops of dimethyl sulphate for 2 h at room temperature. 5.2 mg of the quaternary ammonium salt dissolved in 2 ml dichloromethane (DCM) was treated with two drops of diisopropylethylamine (DIEA). The reaction mixture was stirred at room temperature for 2 h. The solvents then were evaporated and the residue was taken into Ethyl acetate, washed with water and dried over Na₂SO₄. After evaporation of the solvent, the residue was dissolved in 1ml of ethyl acetate and was analysed by GC-MS. A characteristic peak of m/z 125 for the phenyl-vinylsulphone fragment was easily detected. The concentration of the cleaved compound was monitored and compared with an internal standard present in a known quantity; 2.5x10⁻⁶ mol of PVS was observed to be present, which is close to the amount of PVS that was linked to the protein during the modification.

### Addition of Cysteine residues in Albumin and Bovine serum albumin to PVS

Albumin or Bovine serum albumin (Sigma) was dissolved in a mixture of acetonitrile/water. Triethylamine was added to the solution and left under nitrogen for 5 minutes. PVS was then added and the reaction mixture was stirred for only 15 min at room temperature. The PVS-labelled proteins gave a purple product when a portion was reacted with ninhydrin, indicating that free amines were present. After the labelling reaction, the solvent was evaporated at room temperature, and the residue from each protein was precipitated from ether and was recovered by filtration. The solid was washed several times with ethyl acetate and was dried under reduced pressure.

### Thermal elimination of PVS from the Thio-labelled Albumin and BSA

Both samples (PVS thio-labelled albumin and BSA), when injected into the GC-MS as a solution (water/acetonitrile, 75 % ACN) gave distinctive peaks at m/z 125 corresponding to thermally eliminated sulphone. Experiments with PVS labelled albumin and BSA that had been separated by gel electrophoresis, with sodium dodecyl sulphate on a polyacrylamide gel (SDS-PAGE) and recovered using standard methods known in the art, also gave peaks at m/z 125 characteristic of PVS when analysed by GC-MS.

### Treatment of thio-sulphones with aqueous and methanolic sodium hydroxide

PVS labelled proteins are stable under the conditions of SDS-PAGE. It is desirable to be able to analyse proteins by iso-electric focusing, where proteins are separated on an electrophoretically maintained pH gradient. Proteins are separated according to their net charge and focus at the point where the pH leaves the protein with no net charge. Certain proteins are very basic and will focus at a very high pH and may be exposed to basic conditions for several hours. In order to assess whether PVS thiol derivatives are stable under basic conditions, various PVS thio-labelled compounds were incubated for prolonged periods in the presence of 1 M aqueous NaOH and a methanolic solution of 1 M NaOH.

S-(phenyl-ethylsulphonyl)octylthiol in acetonitrile was treated with 1 M aqueous NaOH for 24 h with full recovery of the starting material after that time. When S-(phenyl-ethylsulphonyl)cysteine methyl-ester in acetonitrile was treated with 1 M aqueous NaOH for 24 h, slight cleavage of the sulphone was observed by UV stimulated emission from a Thin Layer Chromatography (TLC) plate after 2 h. But after 24 h, only 9.5 % of the sulphone had cleaved. The rest of the starting material was recovered as shown by the ¹H-NMR. S-(phenyl-ethylsulphonyl)acetylcysteine methyl ester was synthesised by acetylation of S-(Phenyl-ethylsulphonyl)cysteine methyl-ester with acetic anhydride. This compound in acetonitrile was also treated with 1 M aqueous for 24 h. The very slight cleavage of the sulphone has been observed by TLC. But after 24 h, the percentage of the sulphone that was cleaved is only 7 %. The rest of the starting material was recovered and the ¹H-NMR spectrum was recorded. Similar results were obtained from analysis of these compounds in methanolic NaOH. It seems that a free amine in close proximity to the labelled thiol increases cleavage of the PVS-thiol derivative. Since the majority of thiol residues will not be at the N-terminus of a protein, this effect should not be a problem when using sulphones as labelling reagents for proteins. Moreover, in a typical iso-electric focusing separation, the most basic pH is about 12 whereas 1 M NaOH represents a pH of 14.

## Claims

1. A method for characterising an analyte, which method comprises:
(a) providing a compound in which the analyte is attached by a cleavable linker to a reporter group capable of identifying the analyte, wherein the reporter group comprises a mass marker detectable by mass spectrometry, the compound having the following formula: 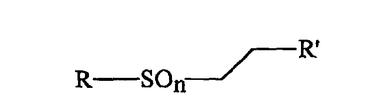 wherein either R comprises the reporter group and R' comprises the analyte, or R comprises the analyte and R' comprises the reporter group, and wherein n is 1 or 2;
(b) cleaving the reporter group from the analyte; and
(c) identifying the reporter group, thereby characterising the analyte.

2. A method according to claim 1, wherein R and/or R' comprise a covalent linkage attaching the analyte and/or reporter group to the cleavable linker.

3. A method according to claim 2, wherein the covalent linkage is independently selected from a -CO-NH- group, an -NH-CO-NH- group, an -NH-CS-NH- group, a -CH₂-NH- group, an -SO₂-NH- group, an -NH-CH₂-CH₂- group, or an -OP(=O)(O)O- group.

4. A method according to any preceding claim, wherein R comprises, between the SOₙ group and the reporter group or analyte, a substituted or unsubstituted aromatic cyclic group, aliphatic cyclic group, or heterocyclic group.

5. A method according to claim 4, wherein R comprises, between the SOₙ group and the reporter group or analyte, a substituted or unsubstituted group selected from phenyl, pyridyl, pyranyl, naphthyl, anthracyl, pyrenyl, or fused ring derivatives or heteroaromatic analogues of the above.

6. A method according to claim 5, wherein the phenyl group is a group having the following formula: 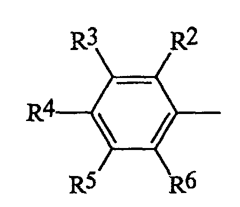 wherein one of R²-R⁶ comprises the reporter group or analyte, and the remaining R²-R⁶ groups are independently selected from a hydrogen, and a substituent, such as a D, F, methyl, methoxy, hydroxy or amino group.

7. A method according to any preceding claim, wherein the R' group comprises a group selected from -S-, -SO-, -NR¹-, and -O- between the C atom that is in the β-position to the SOₙ group, and the reporter group or analyte.

8. A method according to claim 7, wherein the R¹ group is an electron withdrawing group.

9. A method according to claim 8, wherein R¹ is a hydrogen atom, a halogen atom, or a substituent comprising a carbonyl group and/or a halogen atom.

10. A method according to claim 9, wherein R¹ is a fluorine atom, a chlorine atom, a bromine atom, an iodine atom, a trifluoroacetyl group, or a trifluoromethyl acetate group.

11. A method according to claim 1, wherein the compound has the following formula: 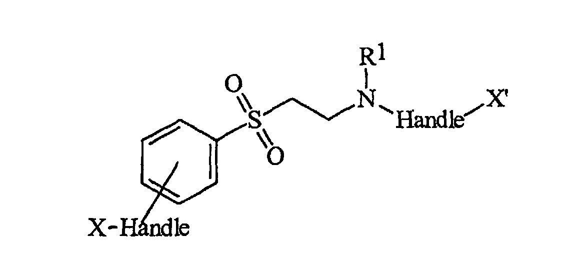 wherein R¹ is an electron withdrawing substituent, X comprises the reporter group and X' comprises the analyte, or X comprises the analyte and X' comprises the reporter group, and each Handle is the same or different, being either a single bond directly attaching the X groups to the phenyl ring and the N atom respectively, or a reactive group capable of attaching the X groups to the phenyl ring and the N atom respectively.

12. A method according to claim 12, wherein R¹ is selected from a hydrogen atom, a halogen atom, or a substituent comprising a carbonyl group and/or a halogen atom.

13. A method according to claim 12 or claim 13, wherein each Handle is independently selected from a -CO-NH- group, an -NH-CO-NH- group, an -NH-CS-NH- group, a -CH₂-NH- group, an -SO₂-NH- group, an -NH-CH₂-CH₂- group, or an -OP(=O)(O)O- group.

14. A method according to any preceding claim, wherein the analyte comprises a biological molecule.

15. A method according to claim 14, wherein the biological molecule is selected from a protein, a polypeptide, an amino acid, a nucleic acid, a nucleic acid base, a pharmaceutical agent or drug, a carbohydrate, a lipid, a natural product and a synthetic compound from an encoded chemical library.

16. A method according to claim 15, wherein the nucleotide, oligonucleotide or nucleic acid is natural, or is modified by modifying a base, sugar and/or backbone of the nucleotide, oligonucleotide or nucleic acid.

17. A method according to claim 15 or claim 16, wherein the analyte is an amino acid or a peptide comprising a cysteine group, and the compound is of the formula: 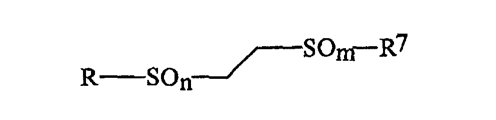 wherein m is 0 or 1 and the S atom attaching R⁷ to the linker is the sulphur atom of the cysteine group, R⁷ being the remainder of the amino acid or polypeptide.

18. A method according to claim 15 or claim 16, wherein the analyte is an amino acid or a peptide, and the compound is of the formula: 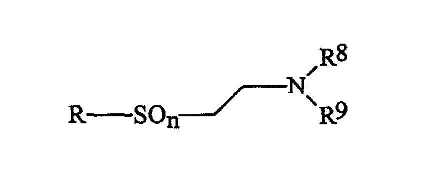 wherein the N atom is the nitrogen atom of an epsilon amino group of a lysine group, or is the nitrogen atom of an N-terminal alpha amino group, R⁸ is selected from H, O or an N-protective group, R⁹ being the remainder of the amino acid or polypeptide.

19. A method according to claim 15 or claim 16, wherein the analyte is an arnino acid or a peptide comprising a serine, threonine and/or tyrosine group, and the compound is of the formula: 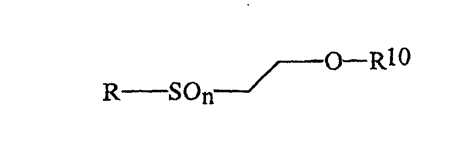 wherein the O atom is the oxygen atom from a hydroxyl group of the serine, threonine or tyrosine group, R¹⁰ being the remainder of the amino acid or polypeptide.

20. A method according to any preceding claim, wherein the mass marker comprises an oligoether or a polyether.

21. A method according to claim 20, wherein the oligoether or polyether is a substituted or unsubstituted oligo- or poly-arylether.

22. A method according to claim 20 or claim 21, wherein the oligoether or polyether comprises one or more fluorine atom or methyl group substituents, or one or more ²H or ¹³C isotopic substituents.

23. A method according to any preceding claim, wherein the mass marker comprises a metal ion-binding moiety.

24. A method according to claim 23, wherein the metal ion-binding moiety is a porphyrin, a crown ether, hexahistidine, or a multidentate ligand.

25. A method according to claim 24, wherein the metal ion-binding moiety is a bidentate ligand or is EDTA.

26. A method according to any of claims 23-25, wherein the metal ion-binding moiety is bound to a monovalent, divalent or trivalent metal ion.

27. A method according to claim 26, wherein the metal ion is a transition metal ion, or a metal ion of group IA, IIA or IIIA of the periodic table.

28. A method according to claim 27, wherein the metal ion is Ni²⁺, Li⁺, Na⁺, K⁺, Mg²⁺, Ca²⁺, Sr²⁺, Ba²⁺, or Al³⁺.

29. A method according to any preceding claim, which method further comprises heating the linker to cleave off the reporter group.

30. A method according to any preceding claim, wherein the method further comprises cleaving off the mass marker in a mass spectrometer.

31. Use of a linker group in the characterisation of an analyte, to attach a reporter group to the analyte, wherein the reporter group comprises a mass marker detectable by mass spectrometry and the linker group is cleavable and has the following formula: 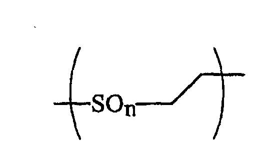 wherein n is 1 or 2.

32. Use according to claim 31, wherein the analyte is as defined in any of claims 14-19.

33. Use according to claim 31 or claim 32, wherein the mass marker is as defined in any of claims 20-28.

34. A compound having the following formula: 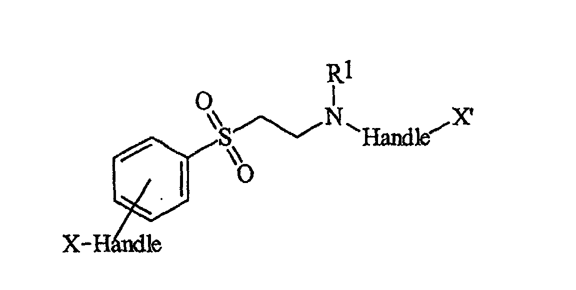 wherein R¹ is an electron withdrawing substituent, X comprises a reporter group and X' comprises the analyte, or X comprises the analyte and X' comprises the reporter group, wherein the reporter group comprises a mass marker detectable by mass spectrometry and each Handle is the same or different, being either a single bond directly attaching the X groups to the phenyl ring and the N atom respectively, or a reactive group capable of attaching the X groups to the phenyl ring and the N atom respectively.

35. A compound according to claim 34, wherein R¹ is selected from a hydrogen atom, a halogen atom, or a substituent comprising a carbonyl group and/or a halogen atom.

36. A compound according to claim 34 or claim 35, wherein each Handle is independently selected from a -CO-NH- group, an -NH-CO-NH- group, an -NH-CS-NH- group, a -CH₂-NH- group, an -SO₂-NH- group, an -NH-CH₂-CH₂- group, or an -OP(=O)(O)O- group.

37. A compound according to any of claims 34-36, wherein the analyte is as defined in any of claims 14-19.

38. A compound according to any of claims 34 to 37, wherein the mass marker is as defined in any of claims 20-28.

## Patentansprüche

1. Verfahren zur Charakterisierung eines Analyten, welches Verfahren Folgendes umfasst:
(a) das Bereitstellen einer Verbindung, in welcher der Analyt durch einen spaltbaren Linker an eine Reportergruppe gebunden ist, die zur Identifizierung des Analyten in der Lage ist, wobei die Reportergruppe einen mittels Massenspektrometrie nachweisbaren Massenmarker umfasst und die Verbindung die folgende Formel besitzt: 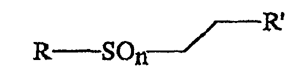 wobei entweder R die Reportergruppe und R' den Analyten umfasst oder R den Analyten und R' die Reportergruppe umfasst und wobei n 1 oder 2 ist;
(b) das Abspalten der Reportergruppe vom Analyten; und
(c) das Identifizieren der Reportergruppe, wodurch der Analyt charakterisiert wird.

2. Verfahren gemäß Anspruch 1, wobei R und/oder R' eine den Analyten und/oder die Reportergruppe an den spaltbaren Linker bindende, kovalente Bindung umfassen.

3. Verfahren gemäß Anspruch 2, wobei die kovalente Bindung unabhängig ausgewählt ist aus einer -CO-NH-Gruppe, einer -NH-CO-NH-Gruppe, einer -NH-CS-NH-Gruppe, einer -CH₂-NH-Gruppe, einer -SO₂-NH-Gruppe, einer -NH-CH₂-CH₂-Gruppe oder einer -OP(=O)(O)O-Gruppe.

4. Verfahren gemäß einem der vorhergehenden Ansprüche, wobei R zwischen der SOn-Gruppe und der Reportergruppe oder dem Analyten eine substituierte oder nicht substituierte aromatische cyclische Gruppe, aliphatische cyclische Gruppe oder heterocyclische Gruppe umfasst.

5. Verfahren gemäß Anspruch 4, wobei R zwischen der SOₙ-Gruppe und der Reportergruppe oder dem Analyten eine substituierte oder nicht substituierte Gruppe umfasst, die ausgewählt ist aus Phenyl-, Pyridyl-, Pyranyl-, Naphthyl-, Anthracyl-, Pyrenyl- oder kondensierten Ring-Derivaten oder heteroaromatischen Analoga des Obenstehenden.

6. Verfahren gemäß Anspruch 5, wobei die Phenylgruppe eine Gruppe mit der folgenden Formel ist: 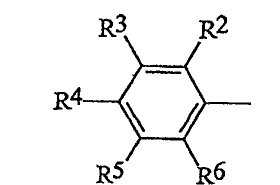 wobei eine von R²-R⁶ die Reportergruppe oder den Analyten umfasst und die verbleibenden R²-R⁶-Gruppen unabhängig ausgewählt sind aus einem Wasserstoff und einem Substituenten, wie z.B. D, F, einer Methyl-, Methoxy-, Hydroxy- oder Aminogruppe.

7. Verfahren gemäß einem der vorhergehenden Ansprüche, wobei die R'-Gruppe eine Gruppe umfasst, die ausgewählt ist aus -S-, -SO-, -NR¹- und -O-, und zwar zwischen dem C-Atom, das sich in der β-Position zur SOₙ-Gruppe befindet, und der Reportergruppe oder dem Analyten.

8. Verfahren gemäß Anspruch 7, wobei die R¹-Gruppe eine elektronenabziehende Gruppe ist.

9. Verfahren gemäß Anspruch 8, wobei R¹ ein Wasserstoffatom, ein Halogenatom oder ein Substituent, umfassend eine Carbonylgruppe und/oder ein Halogenatom, ist.

10. Verfahren gemäß Anspruch 9, wobei R¹ ein Fluoratom, ein Chloratom, ein Bromatom, ein Iodatom, eine Trifluoracetylgruppe oder eine Trifluormethylacetatgruppe ist.

11. Verfahren gemäß Anspruch 1, wobei die Verbindung die folgende Formel besitzt: 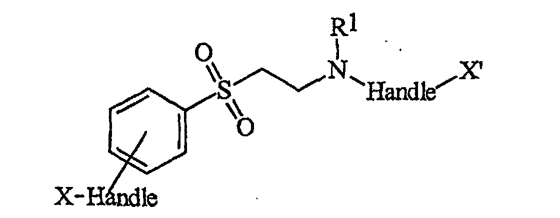 wobei R¹ ein elektronenabziehender Substituent ist, X die Reportergruppe und X' den Analyten umfasst oder X den Analyten und X' die Reportergruppe umfasst und jeder Handle gleich oder verschieden ist, wobei er entweder eine die X-Gruppen direkt an den Phenylring bzw. an das N-Atom bindende Einfachbindung oder eine reaktive Gruppe ist, die zum Binden der X-Gruppen an den Phenylring bzw. an das N-Atom in der Lage ist.

12. Verfahren gemäß Anspruch 12, wobei R¹ ausgewählt ist aus einem Wasserstoffatom, einem Halogenatom oder einem Substituenten, umfassend eine Carbonylgruppe und/oder ein Halogenatom.

13. Verfahren gemäß Anspruch 12 oder Anspruch 13, wobei jeder Handle unabhängig ausgewählt ist aus einer -CO-NH-Gruppe, einer -NH-CO-NH-Gruppe, einer -NH-CS-NH-Gruppe, einer -CH₂-NH-Gruppe, einer -SO₂-NH-Gruppe, einer -NH-CH₂-CH₂-Gruppe oder einer -OP(=O)(O)O-Gruppe.

14. Verfahren gemäß einem der vorhergehenden Ansprüche, wobei der Analyt ein biologisches Molekül umfasst.

15. Verfahren gemäß Anspruch 14, wobei das biologische Molekül ausgewählt ist aus einem Protein, einem Polypeptid, einer Aminosäure, einer Nucleinsäure, einer Nucleinsäurebase, einem pharmazeutischen Wirkstoff oder Arzneimittel, einem Kohlenhydrat, einem Lipid, einem natürlichen Produkt und einer synthetischen Verbindung aus einer codierten chemischen Bibliothek.

16. Verfahren gemäß Anspruch 15, wobei das Nucleotid, das Oligonucleotid oder die Nucleinsäure natürlich ist oder durch Modifikation einer Base, eines Zuckers und/oder eines Grundgerüsts des Nucleotids, des Oligonucleotids oder der Nucleinsäure modifiziert ist.

17. Verfahren gemäß Anspruch 15 oder Anspruch 16, wobei der Analyt eine Aminosäure oder ein Peptid, umfassend eine Cysteingruppe, ist und die Verbindung die Formel 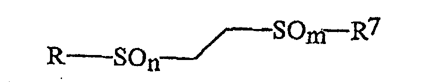 aufweist, wobei m 0 oder 1 ist und das R⁷ an den Linker bindende S-Atom das Schwefelatom der Cysteingruppe ist, wobei R⁷ der Rest der Aminosäure oder des Polypeptids ist.

18. Verfahren gemäß Anspruch 15 oder Anspruch 16, wobei der Analyt eine Aminosäure oder ein Peptid ist und die Verbindung die Formel 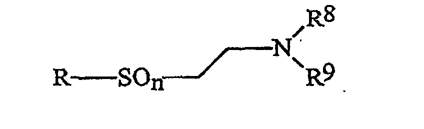 aufweist, wobei das N-Atom das Stickstoffatom einer Epsilon-Aminogruppe einer Lysingruppe oder das Stickstoffatom einer N-terminalen Alpha-Aminogruppe ist, R⁸ ausgewählt ist aus H, O oder einer N-Schutzgruppe, wobei R⁹ der Rest der Aminosäure oder des Polypeptids ist.

19. Verfahren gemäß Anspruch 15 oder Anspruch 16, wobei der Analyt eine Aminosäure oder ein Peptid, umfassend eine Serin-, Threonin- und/oder Tyrosingruppe, ist und die Verbindung die Formel 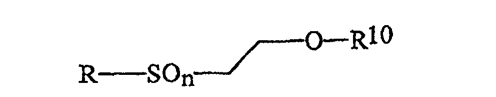 aufweist, wobei das O-Atom das Sauerstoffatom aus einer Hydroxylgruppe der Serin-, Threonin- oder Tyrosingruppe ist, wobei R¹⁰ der Rest der Aminosäure oder des Polypeptids ist.

20. Verfahren gemäß einem der vorhergehenden Ansprüche, wobei der Massenmarker einen Oligoether oder einen Polyether umfasst.

21. Verfahren gemäß Anspruch 20, wobei der Oligoether oder Polyether ein substituierter oder nicht substituierter Oligo- oder Polyarylether ist.

22. Verfahren gemäß Anspruch 20 oder Anspruch 21, wobei der Oligoether oder Polyether einen oder mehrere Fluoratom- oder Methylgruppensubstituenten oder einen oder mehrere ²H- oder ¹³C-Isotopensubstituenten umfasst.

23. Verfahren gemäß einem der vorhergehenden Ansprüche, wobei der Massenmarker eine Metallion-Bindungs-Komponente umfasst.

24. Verfahren gemäß Anspruch 23, wobei die Metallion-Bindungs-Komponente ein Porphyrin, ein Kronenether, Hexahistidin oder ein mehrzähniger Ligand ist.

25. Verfahren gemäß Anspruch 24, wobei die Metallion-Bindungs-Komponente ein zweizähniger Ligand oder EDTA ist.

26. Verfahren gemäß einem der Ansprüche 23-25, wobei die Metallion-Bindungs-Komponente an ein einwertiges, zweiwertiges oder dreiwertiges Metallion gebunden ist.

27. Verfahren gemäß Anspruch 26, wobei das Metallion ein Übergangsmetallion oder ein Metallion der Gruppe IA, IIA oder IIIA des Periodensystems ist.

28. Verfahren gemäß Anspruch 27, wobei das Metallion Ni²⁺, Li⁺, Na⁺, K⁺, Mg²⁺, Ca²⁺, Sr²⁺, Ba²⁺ oder Al³⁺ ist.

29. Verfahren gemäß einem der vorhergehenden Ansprüche, welches Verfahren weiters die Erhitzung des Linkers zwecks Abspaltung der Reportergruppe umfasst.

30. Verfahren gemäß einem der vorhergehenden Ansprüche, wobei das Verfahren weiters die Abspaltung des Massenmarkers in einem Massenspektrometer umfasst.

31. Verwendung einer Linker-Gruppe bei der Charakterisierung eines Analyten, um eine Reportergruppe an einen Analyten zu binden, wobei die Reportergruppe einen durch Massenspektrometrie nachweisbaren Massenmarker umfasst und die Linker-Gruppe spaltbar ist und die folgende Formel besitzt: 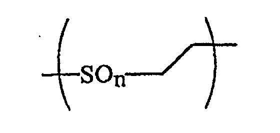 wobei n 1 oder 2 ist.

32. Verwendung gemäß Anspruch 31, wobei der Analyt so ist wie in einem der Ansprüche 14-19 definiert.

33. Verwendung gemäß Anspruch 31 oder Anspruch 32, wobei der Massenmarker so ist wie in einem der Ansprüche 20-28 definiert.

34. Verbindung mit der folgenden Formel: 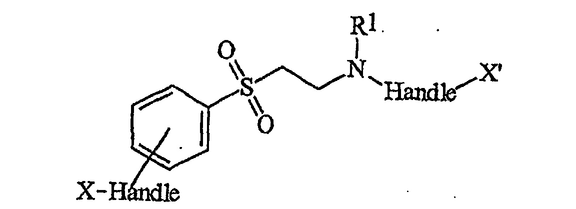 wobei R¹ ein elektronenabziehender Substituent ist, X eine Reportergruppe und X' den Analyten umfasst oder X den Analyten und X' die Reportergruppe umfasst, wobei die Reportergruppe einen durch Massenspektrometrie nachweisbaren Massenmarker umfasst und jeder Handle gleich oder verschieden ist, wobei er entweder eine die X-Gruppen direkt an den Phenylring bzw. an das N-Atom bindende Einfachbindung oder eine reaktive Gruppe ist, die zur Bindung der X-Gruppen an den Phenylring bzw. an das N-Atom in der Lage ist.

35. Verbindung gemäß Anspruch 34, wobei R¹ ausgewählt ist aus einem Wasserstoffatom, einem Halogenatom oder einem Substituenten, umfassend eine Carbonylgruppe und/oder ein Halogenatom.

36. Verbindung gemäß Anspruch 34 oder Anspruch 35, wobei jeder Handle unabhängig ausgewählt ist aus einer -CO-NH-Gruppe, einer -NH-CO-NH-Gruppe, einer -NH-CS-NH-Gruppe, einer -CH₂-NH-Gruppe, einer -SO₂-NH-Gruppe, einer -NH-CH₂-CH₂-Gruppe oder einer -OP(=O)(O)O-Gruppe.

37. Verbindung gemäß einem der Ansprüche 34-36, wobei der Analyt so ist wie in einem der Ansprüche 14-19 definiert.

38. Verbindung gemäß einem der Ansprüche 34 bis 37, wobei der Massenmarker so ist wie in einem der Ansprüche 20-28 definiert.

## Revendications

1. Méthode pour caractériser une substance à analyser, laquelle méthode consiste à :
(a) fournir un composé dans lequel la substance à analyser est reliée par un élément de liaison à clivage à un groupe "sentinelle" capable d'identifier la substance à analyser, où le groupe sentinelle comprend un marqueur de masse détectable par spectrométrie de masse, le composé lui-même ayant la formule suivante : 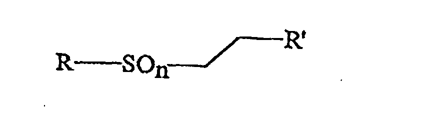 où R comporte le groupe sentinelle et R' comporte la substance à analyser, ou inversement, avec n = 1 ou 2 ;
(b) cliver le groupe sentinelle pour le séparer de la substance à analyser ; et
(c) identifier le groupe sentinelle, caractérisant ainsi la substance à analyser.

2. Méthode selon la revendication 1, où R et/ou R' comportent une liaison covalente reliant la substance à analyser et/ou le groupe sentinelle à l'élément de liaison à clivage.

3. Méthode selon la revendication 2, où la liaison covalente est indépendamment sélectionnée parmi les groupes suivants : -CO-NH-, -NH-CO-NH-, -NH-CS-NH-, -CH₂-NH-, -SO₂-NH-, -NH-CH₂-CH₂, ou -OP(=O) (O) O-.

4. Méthode selon l'une des revendications précédentes, où R comporte, entre le groupe SOₙ et le groupe sentinelle ou la substance à analyser, un groupe cyclique aromatique substitué ou non, un groupe cyclique aliphatique ou un groupe hétérocyclique.

5. Méthode selon la revendication 4, où R comporte, entre le groupe SOₙ et le groupe sentinelle ou la substance à analyser, un groupe substitué ou non sélectionné parmi les
groupes suivants : phényle, pyridyle, pyranyle, naphtyle, anthracyle, pyrényle ou dérivés à composés cycliques fusionnés ou héréto-aromatiques analogues aux précédents.

6. Méthode selon la revendication 5, où le groupe phényle a la formule suivante : 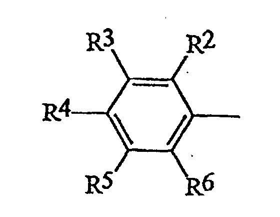 où l'un des groupes R²-R⁶ comprend le groupe sentinelle ou la substance à analyser, les groupes R²-R⁶ restants étant indépendamment sélectionnés parmi un atome d'hydrogène et un substituant, tel qu'un groupe D, F, méthyle, méthoxy, hydroxyle ou aminé.

7. Méthode selon l'une des revendications précédentes, où le groupe R' comprend un groupe sélectionné parmi -S-, -SO-, -NR¹- et -O- entre l'atome de carbone en position β par rapport au groupe SOₙ et le groupe sentinelle ou la substance à analyser.

8. Méthode selon la revendication 7, où le groupe R¹ est un groupe d'extraction électronique.

9. Méthode selon la revendication 8, où R¹ est un atome d'hydrogène, un atome d'halogène ou un substituant comprenant un groupe carbonyle et/ou un atome d'halogène.

10. Méthode selon la revendication 9, où R¹ est un atome de fluor, un atome de chlore, un atome de brome, un atome d'iode, un groupe trifluoracétyle ou un groupe acétate de trifluorométhyle.

11. Méthode selon la revendication 1, où le composé a la formule suivante : 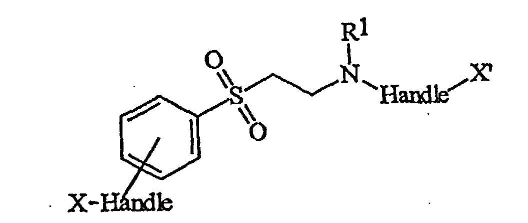 où R¹ est un substituant d'extraction électronique, X comporte le groupe sentinelle et X' la substance à analyser, ou inversement, et chaque "poignée" (*Handle*) est la même ou différente, étant soit une liaison simple reliant directement les groupes X au noyau phényle et à l'atome d'azote, respectivement, soit un groupe réactif capable de relier les groupes X au noyau phényle et à l'atome d'azote, respectivement.

12. Méthode selon la revendication 12, où R¹ est sélectionné parmi un atome d'hydrogène, un atome d'halogène ou un substituant comportant un groupe carbonyle et/ou un atome d'halogène.

13. Méthode selon la revendication 12 ou la revendication 13, où chaque poignée est indépendamment sélectionnée parmi les groupes suivants : -CO-NH-, -NH-CO-NH-, -NH-CS-NH-, -CH₂-NH-, -SO₂-NH-, -NH-CH₂-CH₂, OU-OP(=O) (O) O-.

14. Méthode selon l'une des revendications précédentes, où la substance à analyser est une molécule biologique.

15. Méthode selon la revendication 14, où la molécule biologique est sélectionnée parmi une protéine, un polypeptide, un acide aminé, un acide nucléique, une base d'acide nucléique, un composé pharmaceutique ou un médicament, un sucre, un lipide, une substance naturelle et un composé de synthèse tiré d'une banque chimique codée.

16. Méthode selon la revendication 15, où le nucléotide, l'oligonucléotide ou l'acide nucléique est naturel, ou est modifié en changeant une base, un sucre et/ou le squelette du nucléotide, de l'oligonucléotide ou de l'acide nucléique.

17. Méthode selon la revendication 15 ou la revendication 16, où la substance à analyser est un acide aminé ou un peptide comprenant un résidu cystéine, et le composé lui-même est de formule : 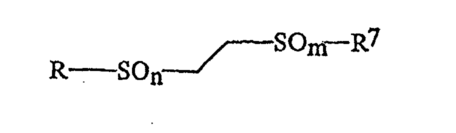 où m est égal à 0 ou 1 et l'atome S reliant R⁷ à l'élément de liaison est l'atome de soufre du résidu cystéine, R⁷ étant le reste de l'acide aminé ou du polypeptide.

18. Méthode selon la revendication 15 ou la revendication 16, où la substance à analyser est un acide aminé ou un peptide, et le composé lui-même est de formule : 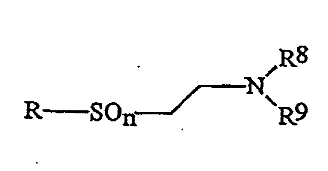 où l'atome N est l'atome d'azote d'un groupe aminé epsilon d'un résidu de lysine ou l'atome d'azote d'un groupe alpha-aminé N-terminal, R⁸ est sélectionnée parmi un atome d'hydrogène d'oxygène ou un groupe N-protecteur et R⁹ est le reste de l'acide aminé ou du polypeptide.

19. Méthode selon la revendication 15 ou la revendication 16, où la substance à analyser est un acide aminé ou un peptide comprenant un résidu sérine, thréonine et/ou tyrosine, et le composé lui-même est de formule : 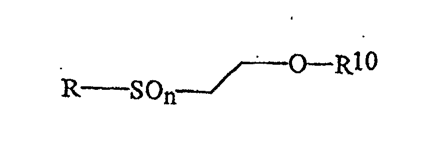 où l'atome O est l'atome d'oxygène d'un groupe hydroxyle du résidu sérine, thréorine ou tyrosine, et R¹⁰ est le reste de l'acide aminé ou du polypeptide.

20. Méthode selon l'une des revendications précédentes, où le marqueur de masse est constitué d'un oligoéther ou d'un polyéther.

21. Méthode selon la revendication 20, où l'oligoéther ou le polyéther est un oligo- ou un polyaryléther, substitué ou non.

22. Méthode selon la revendication 20 ou la revendication 21, où l'oligoéther ou le polyéther comporte au moins un atome de fluor ou un groupe méthyle, ou au moins un substituant isotopique de ²H ou ¹³C.

23. Méthode selon l'une des revendications précédentes, où le marqueur de masse comprend une fraction liant les ions métalliques.

24. Méthode selon la revendication 23, où la fraction liant les ions métalliques est une porphyrine, un crown-éther, une hexahistidine ou un ligand multidentelé.

25. Méthode selon la revendication 24, où la fraction liant les ions métalliques est un ligand bidentelé ou est l'EDTA.

26. Méthode selon l'une des revendications 23 à 25, où la fraction liant les ions métalliques est liée à un ion métallique monovalent, bivalent ou trivalent.

27. Méthode selon la revendication 26, où l'ion métallique est l'ion d'un élément de transition ou l'ion d'un métal des groupes IA, IIA ou IIIA du tableau de Mendeléiev.

28. Méthode selon la revendication 27, où l'ion métallique est Ni²⁺, Li⁺, Na⁺, K⁺, Mg²+, Ca²⁺, Sr²⁺, Ba²⁺ ou Al³⁺.

29. Méthode selon l'une des revendications précédentes, où la méthode comporte en outre le chauffage de l'élément de liaison pour détacher le groupe sentinelle.

30. Méthode selon l'une des revendications précédentes, où la méthode comporte en outre le détachement du marqueur de masse dans un spectromètre de masse.

31. Utilisation d'un groupe de liaison dans la caractérisation d'une substance à analyser, pour fixer un groupe sentinelle à la substance à analyser, où le groupe sentinelle comporte un marqueur de masse détectable par spectrométrie de masse et l'élément de liaison est à clivage et a la formule suivante : 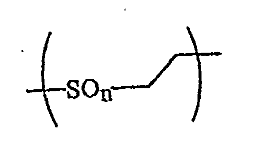 où n = 1 ou 2.

32. Utilisation selon la revendication 31, où la substance à analyser est telle que définie dans les revendications 14 à 19.

33. Utilisation selon la revendication 31 ou la revendication 32, où le marqueur de masse est tel que défini dans les revendications 20 à 28.

34. Composé ayant la formule suivante : 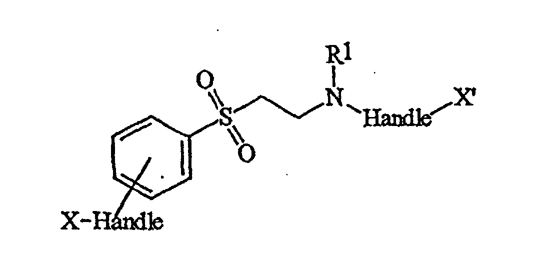 où R¹ est un substituant d'extraction électronique, X un groupe sentinelle et X' la substance à analyser (ou inversement), où le groupe sentinelle comporte un marqueur de masse détectable par spectrométrie de masse et chaque poignée est la même ou différente, étant soit une liaison simple reliant directement les groupes X au noyau phényle et à l'atome d'azote, respectivement, soit un groupe réactif capable de relier les groupes X au noyau phényle et à l'atome d'azote, respectivement.

35. Composé selon la revendication 34, où R¹ est sélectionné parmi un atome d'hydrogène, un atome d'halogène ou un substituant comportant un groupe carbonyle et/ou un atome d'halogène.

36. Composé selon la revendication 34 ou la revendication 35, où chaque poignée est indépendamment sélectionnée parmi les groupes suivants : -CO-NH-, -NH-CO-NH-, -NH-CS-NH-, -CH₂-NH-, -SO₂-NH-, -NH-CH₂-CH₂, OU -OP(=O) (O) O-.

37. Composé selon l'une des revendications 34 à 36, où la substance à analyser est telle que définie dans les revendications 14 à 19.

38. Composé selon l'une des revendications 34 à 37, où le marqueur de masse est tel que défini dans les revendications 20 à 28.
